# EUROPEAN PATENT SPECIFICATION

(11) **EP 0 374 390 B1**
(45) Date of publication and mention of the grant of the patent: **21.06.1995**
(21) Application number: 89118344.4
(22) Date of filing: 03.10.1989
(51) Int. Cl.: A23J 1/20, A61K 35/20, A23C 9/142

(54) **Whey protein composition, a method for producing it and application of the whey protein composition**
Zusammensetzung von Molkeproteinen, Verfahren zur Herstellung und Verwendung dieser Zusammensetzung von Molkenproteinen
Composition de protéines de petit lait, procédé de production et application de la composition de protéines de petit-lait

(30) Priority: 23.12.1988 US 289971
(43) Date of publication of application: 27.06.1990
(73) Proprietor: IMMUNOTECH RESEARCH CORPORATION LTD., Montreal, Quebec H3A 2R7 (CA)
(72) Inventor: Bounos, Gustavo, Montreal, Quebec (CA); Gold, Phil, Quebec H3Y 2S4 (CA)
(74) Representative: Körber, Wolfhart, Dr.rer.nat.

(56) References cited:
- EP-A- 0 022 696
- EP-A- 0 239 722
- WO-A-87/04050
- WO-A-88/08673
- FR-A- 2 296 428
- GB-A- 1 136 309
- GB-A- 1 495 940
- US-A- 4 710 387
- US-A- 4 753 926
- US-A- 4 784 685
- M.K. Horwitt, The Vitamins, 2nd ed., vol.V, 1972, pages 88-96

## Description

The present invention relates to a biologically active whey protein composition comprising a suitable concentration of whey protein concentrate wherein the whey protein concentrate contains proteins which are present in an essentially undenatured state and wherein the biological activity of the whey protein concentrate is dependent on the overall amino acid and small peptides pattern resulting from the contribution of all its protein components, and to a method of producing said whey protein composition comprising the steps of as edfined in the claims 1 to 10

The present invention relates also to the various applications of said whey protein compositions.

It was shown, in controlled experiments, for the first time, that whey protein feeding of mice specifically enhances the immune response to sheep red blood cells (SRBC) and their resistance to pneumococcal infection, inhibits the development of DMH-induced colon cancer and increases tissue glutathione (GSH) levels independently of its nutritional quality.

The present invention shows the correlation between the undenatured conformation of whey protein concentrate (w.p.c.) and host immunoenhancement whereby chemical indices of denaturation are given and the demonstration that the same crucial role of molecular conformation (undenatured state) applies to GSH promotion, which is the other major biological activity of w.p.c.

Equally important is the demonstration that another protein source such as egg white, with the same high cysteine content as w.p.c. does not enhance GSH synthesis, further demonstrating the specificity of w.p.c. with respect to the described biological activity.

The GSH promoting activity of undenatured w.p.c. is sustained over time (3 - 4 months).

Whey and whey protein have been utilized from time immemorable for nutritional purposes. In addition, whey was recommended in folk and ancient medicine for the treatment of various diseases ^{(1,2)} and, in one instance, lifetime feeding of hamsters with a whey protein diet has been shown to promote longevity with no explanation given ^{(3,4)}.

All these conditions appear to be somehow related to changes in glutathione which is a ubiquitous element exerting a protective effect against superoxide radicals and other toxic agents.

### Definitions

a) Whey Protein:
   Whey proteins are the group of milk proteins that remain soluble in "milk serum" or whey after precipitation of caseins at pH 4.6 and 20°C. The whey proteins in cow's milk are:
   - lactalbumins:
      alpha-lactalbumin (α-L), 2-5 % of whole protein, MW: 14,2 Dalton x 10⁻³; serum albumin (SA), 0,7-1,3 % of whole protein, MW: 67 Dalton x 10⁻³;
   - lactoglobulins:
      beta-lactoglobulin (β-L), 7-12 % of whole protein, MW: 18.3 Dalton x 10⁻³; euglobulin, 0,8-1,7 % of whole protein, MW: 180-252 Dalton x 10⁻³;
      pseudoglobulin, 0,6-1,4 % of whole protein, MW: 180-289 Dalton x 10⁻³;
      immuonglobulin, 1,3-2,7 % of whole protein, MW: 150-500 Dalton x 10⁻³;
   - proteose/peptone: 2-6 % of whole protein, MW: 4-20 Dalton x 10⁻³.
   The product of industrial separation of a protein mixture from whey is called "whey protein concentrate" (WPC) or isolate. The major whey proteins in WPC are β-L, α-L, immunoglobulin and SA in order of decreasing amounts.
b) C = casein;
c) SRBC = Sheep red blood cells;
d) PFC = Plaque forming cells (spleen);
   enumeration of PFC in spleen is used to assess the humoral immune response to SRBC injection;
e) GSH = Glutathione (L-γ-glutamyl-L-cysteinylglycine);
f) DMH = 1,2-Dimethylhydrazine;
   DMH 2 HCl = 1,2-Dimethylhydrazine dihydrochloride;
g) The defined formula diets tested varied only in the type of protein;
h) Whey of bovine milk contains approximately 6 g per litre protein, most of the lactose, mineral and water soluble vitamins;
i) wpc = whey protein concentrate;
l) U-Lacp = Undenatured whey protein concentrate;
m) D-Lacp = Denatured whey protein concentrate (Lacprotan-80 from 1) an mark Protein A.S.).

### Historical Background

Dairy products are widely used as a good source of nutrition. In addition claims have been made to the effect that fermented whole milk (yogurt) is beneficial in the management of some types of intestinal infections. Certain dietary regimens based on ill defined natural or cultured dairy products are said to be associated with long life expectancy in some regions of the USSR (Georgia etc.).

Since time immemorable, serum lactis, latin for milk serum or whey, has been administered to the sick for the treatment of numerous ailments. In 1603 Baricelli ⁽¹⁾ reported on the therapeutic use of cow or goat milk serum, sometimes mixed with honey or herbs. The spectrum of illnesses treated with whey included jaundice, infected lesions of the skin, those of the genito-urinary tract with purulent secretions, gonorrhea, epilepsy, quartan fever and other febrile states of different origins. Indeed, the common denominator of most of these illnesses appears to be a septic condition. Although physicians of both Ancient Times and of the Middle Ages agreed that whey treatment should be carried out over a period of several days, a difference of opinion appeared to exist concerning the daily amount prescribed. Thus, Galen, Hippocrates and Dioscoride insisted on a minimum daily amount of two 12 oz latin libras, and up to five libras a day according to gastric tolerance. This would represent between 1 to 2 liters of whey a day. Baricelli, on the other hand, reflecting the trend of his times, limited the amount prescribed to one libra a day, given in fractionated doses on an empty stomach.

Since then, numerous articles published in Europe throughout the 17th, 18th, and 19th centuries have advocated the therapeutic use of whey ⁽²⁾. In an Italian textbook published in the middle of the 19th century ⁽¹⁴⁾, at the dawn of scientific medicine, an interesting distinction is made between whole milk and milk serum. Milk is recommended firstly as a nutrient especially in patients with strictures of the gastrointestinal tract. In this respect the author emphasizes that the benefits of the then popular "milk therapy" of cachexia and tuberculosis are due only to the nutritional property of milk. Secondly, milk was prescribed in the treatment of poisoning because milk components would presumably neutralize ingested toxic material.

Thirdly, milk therapy was suggested for the alleged capacity of this fluid to coat and sooth ulcers of the gastrointestinal tract. Milk serum, on the other hand, was advocated in the treatment of pneumonitis, acute inflammatory diseases of the intestines and urogenital tract, in spite of its recognized lower nutritional quality. Finally the author emphasized the ineffectiveness of whey in the treatment of disorders of the nervous system ⁽¹⁴⁾.

The prime difference between whey (serum lactis) and whole milk is the near absence in the former of the caseins, the casein-bound calcium and phosphate, most of the fat an the fat soluble vitamins. The actual concentration in whey of "whey proteins" is usually similar to that in milk. Hence quantitative differences between whey and milk could not be construed to represent a key factor in the alleged therapeutic effect of whey treatment because, if any, they imply the lack, in whey, of some important nutrients.

Some previously collected data ⁽⁵⁻¹⁰⁾ of the present inventors provide a scientific background to the presumed benefit of intensive treatment with "serum lactis". The importance of the characteristic amino acid in peptide of profile of whey protein concentrate in the immune enhancing effect of the whey protein concentration (WPC) has been shown. The caseins represent 80% of the total protein content of cows milk while WPC is only 20%. Hence, it is conceivable that it is the separation of WPC from the caseins in whey which represents the crucial qualitative change, since this would render the amino acid profile of whey proteins unaltered by that of the caseins, once the digestive process has released free amino acids from all ingested proteins.

### Methods to enhance humoral immune response to sheep red blood cells and the resistance to pneumococcal infection:

### Description of prior art:

An imposing number of publications deal with the association of nutritional deficiencies, including protein energy malnutrition, and infection in the human and animal host. For example, mice fed insufficient amounts of protein, exhibit less growth or even weight loss and increased susceptibility to infection by Staphylococcus aureus⁽¹⁵⁾. Some studies deal with the influence of the type of protein in nutritionally similar and adequate diets on the immune response. More specially in full term babies casein or whey predominant formulas exhibit comparable nutritional efficiency ⁽¹⁶⁾. On the other hand, various investigators have reported that nitrogen retention and growth are greater ⁽¹⁷⁾ serum protein is higher ⁽¹⁸⁾ and metabolic acidosis is less severe ^{(18, 19)} in whey formula fed very low-birth-weight infants than in those fed the casein formula. No incidence of diarrhea was reported during whey-protein feeding ⁽¹⁷⁻¹⁹⁾. Examples in FR-A-2296428 clearly indicate that the whey protein formula is more digestible and nutritionally effective as it promotes growth in malnourished, low weight, diarrheic infants as demonstrated by: a) cessation of diarrhea present from birth in a 6 month old infant and a rapid reversal of unspecified signs of "protein calories malnutrition" (page 6, 1. 5). b) Cessation of "recurrent bouts of intestinal infections", resistant to antibiotics, in a 3 month old infant (page 6, line 15). c) Examples 1, 4 and 5 illustrate therapy of anemia, growth support and treatment of hyperphenylalaninemia respectively. d) Cessation of diarrhea in a 1 month old baby and of malnutrition with return to a positive nitrogen balance (p 7.10). Claim 5, of the FR-A-2296428 advocates "prevent and heal digestive problems, metabolic diseases, intestinal infection and as a cosmetic product in the area of skin treatment". It is quite apparent that the terms "intestinal infection" and "diaarhea" are used interchangeably. In the FR-A-2296428 no reference is made to the immune system. Immunological assays were not carried out, nor even a generic nonsubstantiated claim of improved intestinal or systemic immunity was made. Post-treatment assessment of intestinal flora was not reported.

The immune system was not investigated in FR-A-2296428 and the statement "increased resistance to intestinal infection" based on cessation of diarrhea cannot be construed to represent specific immunoenhancement by whey protein formula independently of its good nutritional quality.

Although no indication is given in FR-A-2298428 with regard to the type of alimentation prior to whey formula feeding it is apparent that the babies were in a state of severe malnutrition and that the improve- ment in growth reported following whey formula feeding was conceivably associated with increased serum albumin levels.

In fact serum prealbumin is a sensitive measure of protein and caloric adequacy in premature infants ⁽²⁰⁾. The effect of hypoalbuminemia on intestinal fluid absorption and diarrhea has been recognized since Starling's original work on fluid absorption ^{(21, 22)}. In addition to this osmotic-type diarrhea, malnutrition, especially protein malnutrition, can produce mucosal atrophy as the intestinal epithelium is primarily fed by luminal nutrients ⁽²³⁾. Malnutrition leads to an absolute decrease in intestinal mucin ⁽²⁴⁾. Impaired capacity to maintain mucosal mucin content and barrier is considered to be a cause of diarrhea by reducing intestinal resistance to enteric infections ⁽²⁴⁾ and to pancreatic proteases ^{(25, 26)}.

Finally, the possibility that the intestinal mucosa of newborn mammals could be temporarily permeable to intact immunoglobulins from mother's milk, as indicated in WO87/04050 is unrelated to the present study. Said document reports a method for concentrating from whey a product containing immunologically active (anti- gen binding) immunoglobulin [Ig] that, when fed to newborn calves at the very high concentration of 7 % of total solids, provides a substantial transfer of natural passive immunity as evidenced by blood I_{g} levels and increased resistance to infections. This temporary increase in passive immunity is associated in the WO87/04050 with initiation of the active immune system of the animal, but does not prove a cause-effect relationship between passive immunity and the development of active immunity.

Traditional medicine and several studies have indicated that lactobacilli constitute one of the dominant groups of the intestinal flora and that the adverse effect of putrefactive organisms could be minimized by maintaining adequate lactobacillus flora in the gut ⁽²⁷⁾. This consideration has contributed to the interest in the therapeutic effect of lactobacilli and dairy products fermented with lactic acid bacteria on various gastrointestinal disorders. In addition, it has been recently shown that in mice drinking fermented milks with Lactobacillus casei and Lactobacillus acidophilus the systemic immune response to SRBC was enhanced. Such enhancement of the immune response was attributed by the authors to the substances produced by these organisms during the fermentation process, such as some metabolites and casein peptides, bacterial enzymes and/or by substances contained in the cell wall of the lactobacilli ⁽²⁸⁾. It is impossible to separate in this model the relative role of bacteria from that of the milk component.

### MECHANISM RESPSONSIBLE FOR THE IMMUNOENHANCING EFFECT OF WHEY PROTEIN CONCENTRATE IN DIET

Over the past few years attempts have been made to identify the changes induced by dietary protein type which might directly or indirectly affect the humoral immune responsiveness. In mice not challenged with an immunogenic stimulus, the type of protein in the diet was found to have little or no effect on a variety of parameters examined. Thus, body growth, food consumption, serum protein, minerals and trace metals, circulating leukocytes and more specifically, the genesis of bone marrow B lymphocytes were all within normal limits ⁽⁵⁻⁹⁾.

The search for the possible mechanism of immunoenhancement by whey protein feeding has revealed to us the provocative possibility that whey protein concentrate may contribute to a broader biological effect of a protective nature involving susceptibility to cancer and general detoxification of environmental agents.The present studies have shown that the observed enhancement of the immune response is associated with greater production of splenix glutathione in immunized mice fed whey protein concentrate in comparison to mice fed casein, cysteine enriched casein or egg white protein in similar dietary concentration. The efficiency of dietary cysteine in inducing supernormal glutathione levels is greater when it is delivered in the whey protein than as free cysteine or within the egg white protein. Glutathione was found at higher levels in the heart and liver of whey protein fed old mice in comparison to mice fed the coresponding casein diet, the egg white protein or Purina Mouse Chow.

### METHODS TO INCREASE TISSUE GLUTATHIONE

### Description of the prior art

Glutathione is a ubiquitous tripeptide thiol (L-γ-glutamyl-L-cysteinylglycine) with a broad range of vital functions that include detoxification of xenobiotics and protection of cells against oxygen intermediates and free radicals, by-products of oxygen-requiring metabolism ⁽³¹⁾ . Modulation of intracellular glutathione affects the proliferative immune response of lymphocytes which may be inhibited by oxidative injury ⁽³²⁾ . Glutathione protects the cells against radiation and alkylating agents . Age-related or experimentally induced glutathione depletion in the lens is associated with cataract formation ⁽³³⁾ . Oxidative DNA damage is rapidly and effectively repaired. The human body is continually repairing oxidized DNA. A small fraction of unrepaired lesions, however, could cause permanent changes in DNA and might be a major contributor to old age diseases and cancer⁽³⁴⁾. Indeed, several age associated diseases may be induced by free radicals . It appears that whereas data on age-related changes in tissue vitamin E and other antioxidants are, at best, contradictory ⁽³⁵⁾, the tissue glutathione levels are more consistently reported to decline with old age in laboratory animals ⁽³⁶⁾ and man ⁽³⁷⁾ .

For these reasons there has been interest in the factors that influence intracellular glutathione synthesis and especially in ways of increasing cellular levels of glutathione.

Glutathione is composed of three amino acids: glutamic acid, glycine and cysteine. Availability of cysteine is a limiting factor in the synthesis of glutathione ⁽³⁸⁾ . Cysteine is derived from dietary protein and by trans-sulfuration from methionine in the liver. Various methods have been tried in order to increase cellular level of glutathione. Administration of free cysteine is not an ideal method because this amino acid is rapidly oxidized, toxic and may actually cause glutathione depletion ⁽³⁹⁾. Similar problems have been encountered with i.p. injection of N-acetyl cysteine to rats, although oral administration of this compound appeared to prevent paracetamol-induced glutathione depletion ⁽³⁹⁾. Administration of compounds that are transported and converted intracellularly into cysteine, such as L-2 oxothiazolidine-4-carboxylate are useful in increasing cellular glutathione ⁽⁴⁰⁾ acting as an intracellular delivery system for cysteine. Hepatic glutathione doubled four hours after injection, returned to normal 8 hours later but was below normal after 16 hours⁽⁴⁰⁾. Another approach for increasing tissue glutathione levels was found in s.c. injection of γ-glutamylcyst(e)ine in mice: glutathione increased in the kidney by about 55%, 40-60 minutes after injection, returning to near control values 2 hours later⁽⁴¹⁾. The administered compound is transported intact and serves as a substrate for glutathione synthetase. It was also reported that about 2 hours after i.p. administration of γ-glutamyl cysteinyl-glycyl monomethyl (or monoethyl) ester to mice, the liver and kidney glutathione levels were doubled, with return to normal values after 8 hours . Similar increases in glutathione tissue levels were attained by Meister by administering an alkyl monoester of glutathione (US-A-4,784,685), to mice. Such esters are transported into tissue cells, and are deesterified within the cells, thus leading to increased cellular levels of glutathione. The kinetics of tissue glutathione increments attained with this method are similar to those described following i-p. injection of methyl or ethyl esters of glutathione. The effectiveness of these methods has been clearly demonstrated in acute experiments (US-A-4,784,685); in mice treated with L-2-oxothiazolidine-4-carboxylate the expected drop in glutathione tissue level subsequent to acetaminophen injection, was replaced by an actual increase in tissue glutathione values and survival. Other methods to increase tissue glutathione levels are based on the "overshoot" of glutathione concentration, following depletion by diethylmaleate or BSO. These studies were done in vitro on murine cell lines . Also pre-exposure of rats to hypoxia was found to increase lung glutathione .

The administration of glutathione itself is of little consequence on tissue glutathione levels, because it apparently cannot be transported intact across the cell membrane .

Some of said methods of increasing intracellular levels of glutathione concentration are either toxic or dangerous owing to the risks related to the initial phase of glutathione depletion. The methods involving the use of γ-glutamylcyst(e)ine, athiazolidine or glutathione esters (US-A-4,784,685) offer an interesting possibility for short term intervention.

However, their long term effectiveness in producing sustained elevation of cellular glutathione has not been shown, nor has the possible toxicity of their long term use been disproved. Indeed, glutathione and glutathione disulfide were found to be positive in the most commonly used short term tests for carcinogenicity and mutagenicity . Relevant to our invention are recent data indicating specifically that a lack of the GSH precursor, cysteine, rather than a decrease in biosynthetic enzyme activities is responsible for the deficiency of GSH noted in aging animals⁽⁴³⁾. Similarly, the fall in cytosolic GSH in the liver of the chronic ethanol fed rats does not appear to be caused by a limitation in the capacity of γ-glutamylcysteine synthetase activity ⁽⁴⁴⁾.

### METHODS TO INHIBIT THE GROWTH OF CHEMICALLY INDUCED COLON CANCER

### Prior Art

Dietary protein deficiency has been found to reduce the incidence of spontaneous ⁽⁴⁶⁾ or transplanted ^{(46,47)} tumors. Most of the definitive studies concerning protein and cancer have utilized protein underfeeding. Although some evidence indicates that the higher the protein intake, the greater the tumor incidence^{(48,49)} , data concerning the effect of raising protein intake on carcinogenesis and tumor development are not definitive⁽⁵⁰⁾. Studies have focused on the quantity of protein and its amino acid supply rather than its source ⁽⁵⁰⁾. Only a few data are available on the effect of protein type in nutritionally adequate and similar diets on the development of tumors. Specifically our invention deals with the effect of undenatured dietary bovine whey protein concentrate in a nutritionally adequate diet on the development of DMH-induced colon cancer in mice.

Jacquet et al⁽⁵¹⁾ reported that feeding milk retarded on the average by a factor of 0.4 tumor growth in rats implanted with epithelioma T8. This is consistent with some epidemiological studies showing that consumption of milk or dairy products may reduce the risk of cancer ^{(52,53)}. In mice inoculated with Ehrlich ascites tumor cells, feeding yogurt reduced the number of tumor cells by a factor of 0.2-0.28 ⁽⁵⁴⁾. It was also reported that mice fed a milk protein formula diet, exhibited inhibition of tumor volume by a factor of 0.2 to 0.7, following s.c. injection of DMH-induced colon tumor cells in comparison to mice fed other types of protein ⁽⁵⁵⁾. A comparable degree of tumor inhibition was noted in milk protein fed mice injected s.c. with herpes virus transformed cells ⁽⁵⁶⁾. However, in another article, submitted several months later, the same group of authors reported results "...different from those expected in light of our previous findings". Milk protein feeding did not inhibit tumor growth in the same strain of mice injected with DMH ⁽⁵⁷⁾. The previously reported anti-cancer biological property of dietary milk proteins was absent, in spite of the preservation of their good nutritional quality ⁽⁵⁷⁾. The authors provide no explanation of the apparent contradiction.

Various types of cheeses and yogurt were recently found to suppress the growth of several experimental tumors in mice in proportion to the duration of feeding. The tumor size was reduced by a factor of 0.17 to 0.70 depending on the type of tumor ⁽⁵⁸⁾.

DMH-induced colon tumors appear to be similar to those found in humans as far as type of lesions and chemotherapeutic response characteristics are concerned ^{(59,60)}.

About 80% of the proteins in bovine milk are caseins and the remaining 20% are whey proteins ^{(61,62)}. In addition, using the traditional process of preparing casein, the amount of whey protein co-precipitated along with the casein varies from about 40 to 60% of the total amount of whey protein present in the milk ⁽⁶³⁾. Therefore it is conceivable that the minor anti-cancer effect seen with casein could be due to the relatively (to caseins) small amount of whey protein co-precipitated with it.

The GB-A-1495940 (Willadsen) shows an antileukemic effect in mice of a fraction of whey protein presumably of bovine origin. Mice inoculated with leukemia cells were injected i.p. with the test material in an amount that I estimate to be around 0.04 mg/protein per day (6000 - 20,000 mol. wt. representing about 60% of total whey protein) for 9 days.

The only "control" cancer-ineffective proteins used in GB-A-1495940 were the whey fraction less than 6000 mol. wt. or more than 20,000, i.e. the serum albumin (66,000 mol. wt.) and immunoglobulin (153,000 to 1000,000). Most allergenic proteins have a molecular weight of between 15,000 and 40,000 daltons. The major allergen of cow's milk is β-lactoglubulin (MW 18,300) ⁽⁶⁴⁾. Hence, the whey protein fraction shown to have this antileukemic effect (mol. wt. 20,000 - 6000) would include β-lactoglobulin. It is quite conceivable that the anti-cancer effect described in the GB-A-1495940 is the result of immunomodulation by foreign protein antigenic stimulation.

A human whey protein component, lactoferrin, was found to exert, depending on the culture conditions, either a mild inhibitory effect or a stimulatory effect on the growth of a human colon adenocarcinoma cell line in vitro ⁽⁶⁵⁾. This particular protein, present in human milk, is either totally absent or found only in trace amounts in bovine whey ⁽⁶⁶⁾.

### Milk processing and whey preparation

Immediately after milking, the milk is cooled to 4°C and kept in a cooling tank for delivery to the cheese factory. The precipitation of the curd is obtained by reducing the pH to about 4.6 with lactic acid initially at 20°C. Following the addition of rennet (normally three ounces/1000 pounds (= 85g/453.6kg) of milk), the temperature is raised to about 30°C for 20 minutes to promote expulsion of whey from the curd, allowing the agitation in the vat to resolve at low speed.

When sufficient quantity of whey is obtained, the product remaining in the vat is pasteurized in the standard fashion to obtain reduction of bacteria and agitated at high speed for cheese production. The whey is then irradiated with a source of gamma-irradiation. The radiation dose will vary from 5 to 15 kGy according to bacteria content of the whey, to reach equivalent antibacterial effect of standard pasteurization with minimal protein denaturation (measured by changes in soluble protein, i.e. protein concentration in whey before and after treatment).

Accordingly, an object of the invention is to provide a whey protein composition with a specific biological activity which is not related to the already known nutritional quality of this protein and which should be independent of specific genetic or hormonal influences to enhance cellular glutathione content, humoral immune response (PFC) to sheep red blood cells, resistance to pneumo-coccal infection, resistance to dimethyl-hydrazine induced colon carcinoma and to delay the mortality from diseases of aging.

It is an object of the invention to provide a whey protein composition which can be used for human or animal nutrition, for example as substitutes or supplements, for prophylaxis and/or therapeutic purposes.

Another object of the invention is to provide a method for preparing undenatured WPC through ultrafiltration.

Another object of the invention is to provide a way to increase the intracellular levels of glutathione by oral administration of the cysteine precursor as contained in the undenatured whey protein concentrate of bovine origin. Similar amounts of cysteine given as free cysteine are ineffective. On the other hand the same amounts of another protein such as egg white, with an identical high cysteine content, were found to be ineffective in raising tissue glutathione levels. Hence, the specific whey protein concentrate physico-chemical composition, in its undenatured form (i.e. the position of cysteine in relation to the primary, secondary and tertiary structure of the protein), appears to be a crucial factor in the bioavailability of cysteine as a precursor for intracellular synthesis of glutathione.

Another object of the invention is to provide a method for incresing cellular levels of glutathione without exposure to the toxic effects associated with other known methods: the long term ingestion of bovine whey protein concentrate has been shown to be non-toxic in mice, hamsters and humans.

A further object of the invention is to provide a sustained elevation of tissue glutathione for any purpose for which a prophylactic long term elevation of cellular glutathione levels is desired in the prior art, such as for cellular protection against free radicals, foreign hazardous compounds, drug detoxification, radiation, immunodeficiency states etc.

These problems are solved by a whey protein compsition comprising a suitable concentration of whey protein concentrate wherein the whey protein concentrate contains proteins which are present in an essentially underatured state and wherein the biological activity of the whey protein concentrate is dependent on the overall amino acid and small peptides pattern resulting from the contribution of all its protein components.

The invention is further developed by the subclaims. Important is that WPC is bovine and/or sheep and/or human whey protein concentrate.

According to the invention there is provided a method of producing a whey protein concentrate composition according to claims 1 to 3

The whey protein composition produced according to these claims and characterized in claims 4-10 can be used in accordance with the invention as claimed in claims 11 to 18.

### Our studies

The interest in the effect of amino acid intake upon the immune system was prompted by an observation made several years ago.Mice were fed a defined formula diet containing a free amino acid mixture duplicating casein. Another group of mice was fed a similar diet but with moderate restriction of phenylalanine and tyrosine compensated by a corresponding increment in the non-essential amino acid mixture. The second group of mice gained weight at the same rate as the mice fed the casein equivalent mixture or Purina mouse chow. However, when challenged with sheep red blood cells, these mice produced more antibodies and plaque forming cells against sheep red blood cells than Purina or casein equivalent-fed mice.

A new concept thus emerged, namely, that changes in the amino acid profile of the diet can influence the immune response independently of any systemic effect on the nutritional status of the host. But, more importantly, changes in the amino acid profile, i.e. protein type, could conceivably enhance the humoral immune response beyond that which had traditionally been considered to represent a "normal" response.

The effect on the immune response of different types of proteins in nutritionally adequate and similar diets was assessed. Mice fed formula diets containing 20% or 28% whey protein pancreatic hydrolysate (LAD, Nestle) were found to produce more plaque forming cells to sheep red blood cells than mice fed Purina mouse chow containing about 22% protein from various sources and of similar nutritional efficiency. The immune enhancing effect of LAD was maximal at 20% concentration.

A 20 g net protein/100 g diet provides a good method to assess the effect of protein type on the immune system. At this level most protein supplies the minimum daily requirement of all indispensible amino acids for the growing mouse ⁽¹¹⁻¹³⁾and this is important because the amino acid adequacy is not the variable under investigation.

In subsequent studies the effect of dietary whey protein concentrate (WPC) to that of other purified proteins in formula diets of similar nutritional efficiency was compared. The effect of graded amounts of dietary WPC, casein (C), soy (S), wheat (W), protein and Purina rodent chow (stock diet) on the immune responsiveness of C3H/HeN mice has been investigated by measuring the specific humoral immune response to sheep red blood cells (SRBC), and horse red blood cells (HRBC). The nutritional efficiency of these diets was normal and similar. The immune response of mice fed the WPC diets, was found to be almost five times higher than that of mice fed the corresponding C diets. The humoral immune response of mice fed C, S, and W diets was substantially lower than that of mice fed stock diet, whereas that of mice fed L (WPC) diet was higher. The above-described immune effect of all tested proteins was obtained at 20 g/100 g concentration with no further increments with 30- and 40 g/100 g protein in the diet.

Because the whey protein concentrate was tested in comparison to a limited number of proteins, we could not ascertain at that time whether the enhancement of the humoral immunme response observed in five (5) unrelated strains of mice fed a whey protein diet, was due to a real immunoenhancement, in absolute terms, by whey protein feeding or immuno-depression by the other food proteins tested.

Indeed, it can be stated, in retrospect, that these few purified food proteins (casein, soy and wheat) used as "control" for the whey protein mixture were immunosuppressive when compared to all of the other purified food proteins subsequently tested, though nutritionally adequate and similar at 20% concentration in diet.

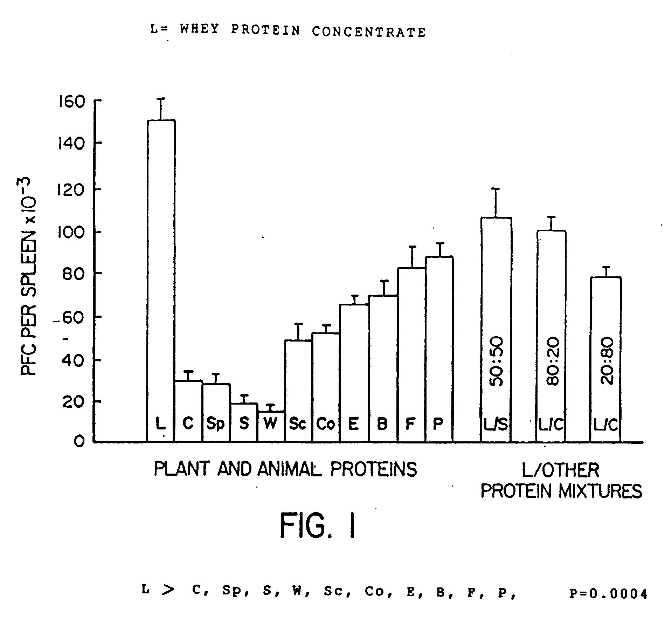

In fact, it was subsequently tested whey protein against most commercially available purified food proteins (casein, soy, wheat, corn, egg white, beef, fish protein, gamma globulin, beta-lactoglobulin, alpha-lactalbumin, serum albumin, spirulina maxima or scenedesmus algae protein) and found that indeed mice fed whey protein concentrate exhibit the highest immune response to foreign antigen (SRBC) (Figure 1).

These proteins are nutritionally similar and adequate at the 20 g/100 g diet concentration (see Table 1 and Table 2).

It was concluded that our newly discovered immune enhancing biological activity of whey protein concentrate is not related to the already known nutritional quality of this protein which is primarily based on digestibility and amino acid content. In fact, the nutritional property of whey protein concentrate at 20 g protein per 100 g diet concentration as used in our experiment is similar to that of the other proteins tested.

### MATERIALS AND METHODS

A suitable source of whey protein concentrate is the material known by the trade mark PROMOD, which is a protein supplement provided in powder form by Ross Laboratories, a Division of Abbott Laboratories, U.S.A. This is a concentrated source of high quality protein which is useful for providing extra protein to persons having increased protein needs, or those who are unable to meet their protein needs with their normal diet. It contains whey protein concentrate and soy lecithin. It has the following nutrients:

| NUTRIENTS | PER 5 G PROTEIN (ONE SCOOP) |
|---|---|
| Protein | 5.0 g |
| Fat | Does not exceed 0.60 g |
| Carbohydrate | Does not exceed 0.67 g |
| Water | Does not exceed 0.60 g |
| Calcium | Does not exceed 23 mg (1.15 mEq) |
| Sodium | Does not exceed 13 mg (0.57 mEq) |
| Potassium | Does not exceed 65 mg (1.66 mEq) |
| Phosphorus | Does not exceed 22 mg |
| Calories | 28 |

It has the following typical amino acid composition per 100 g protein. 100 g PROMOD protein yields approximately 105 g of amino acids.

### TYPICAL AMINO ACID COMPOSITION per 100 g Protein

Essential Amino Acids:
Histidine, 1.9 g;
Isoleucine, 6.2 g;
Leucine, 10.8 g;
Lysine, 9.3 g;
Methionine, 2.2 g;
Phenylalanine, 3.6 g;
Threonine, 7.3 g;
Tryptophan, 1.9 g;
Valine, 6.0 g.

Non-Essential Amino Acids:
Alanine, 5.3 g;
Arginine, 2.6 g;
Aspartic Acid, 11.2 g;
Cysteine, 2.6 g;
Glutamic Acid, 18.2 g;
Glycine, 2.1 g;
Proline, 6.5 g;
Serine, 5.6 g;
Tyrosine, 3.4 g.

### Diets used in our studies:

Diets are prepared in the following way: 20 g of selected pure protein, 56 g of product 80056 protein free diet powder containing corn syrup, corn oil, tapioca starch, vitamins and minerals (Mead-Johnson Co. Inc., U.S.A.), 18 g cornstarch, 2 g wheat bran; 0.05 g Nutramigen vit-iron premix (Bristol-Myers, Ontario, Canada), 2.65 g KCl; 0.84 g NaCl. The carbohydrate and lipid components of our formula diets were the same. The only variable in the various purified diets was the type of protein (20 g protein/100 g diet). At this concentration in diet all the different proteins tested provided the daily requirements of essential amino acids for the growing mouse ⁽¹¹⁾. Vitamins and minerals were the same in each set of experiments and were added in the amount necessary to provide daily requirements for the growing mouse ^{(5-8,13 )}. Table 1 indicates the variation in suggested vitamin requirements for mouse diets and their contents in some of our formulations. Therefore all the formula diets used in our experiments were designed to provide adequate nutrition as demonstrated by normal body growth, serum protein ⁽⁵⁻⁹⁾ and by the absence of hair loss, dermatitis, cataract, ataxia, fatty liver etc. The latter symptoms were of course present in very old mice and were related to the aging process.

### Animals

Male C3H/HeJ mice were obtained from Jackson Laboratories (Bar Harbor, Maine) at 7 weeks of age.

### Immunization for plaque assays

The diet-fed mice were immunized by an intravenous injection of 5 x 10⁶ washed sheep red blood cells obtained weekly from Institut Armand-Frappier, Laval des Rapides, Quebec, Canada.

### Plaque forming cell (PFC) assay

The method used for assaying IgM plaque forming cells was essentially the one described by Cunningham and Szenberg (67), with certain minor modifications. Spleen cell suspensions were prepared by gently tamping the spleen through a 50-mesh stainless steel screen, and collecting the cells in balanced salt solution (BSS) supplemented with 10% heat-inactivated calf serum (Grand Island Biological Company, Montreal, Quebec, Canada). The spleen cells were washed and made up to 15 ml with BSS. Sheep red blood cells were washed twice and made up to a 20% concentration. Guinea pig serum (Grand Island Biological Company, Montreal, Quebec, Canada) as a source of complement was diluted 1/15 with BSS. All stock solutions were kept on ice water until used. The test consisted of mixing 0.05 ml of spleen cells, 0.15 ml of sheep red blood cells and 0.75 ml of the complement solution in a test tube at 37°C. The whole mixture was immediately withdrawn and put into slide chambers, sealed with warm paraffin wax, and incubated at 37°C for 45 to 60 min. The number of plaque forming cells was counted and their total number per spleen estimated by multiplying the number of plaque forming cells in each sample (0.05 ml spleen cells) by 300. The values are expressed per total organ rather than per 10⁶ spleen cells, since this appears to reflect more accurately the functional status of the spleen per se.

Mice were assayed for the plaque forming cell response to sheep red blood cells normally on the fifth day after immunization when the response was shown to peak or, in the kinetic study, on days 3, 4, 5 and 6 post-immunization.

### Statistics

The mean plaque forming cell values were compared among the dietary groups using either Student's-test, when two groups were being compared, or the analysis of variance (ANOVA) for more than two groups. Because of the heterogeneity of variances among groups, the adjustment given by Brown and Forsythe was used.

### Spleen glutathione content

Ninety milligrams of mouse spleen were weighed using a Mettler PM-300 balance and samples varied from 90 mg by less than 5 mg ( 5%). The samples were then homogenized in 5-sulfosalicylic acid (5% w/v). Homogenates were centrifuged for 5 min in a microfuge at 10,000 x g. The assay was carried out using the supernatants on the same day according to the methods of Anderson (42). Values are expressed as »mol per g/wet tissue.

### Buthionine sulfoximine experiments

In some experiments, following three weeks of whey protein feeding and one day prior to immunization with sheep red blood cells, mice were injected i.p. with 450 mg/kg of buthionine sulfoximine (BSO) (S-[n-butyl] homocysteine sulfoximine), a specific inhibitor of γ-glutamylcysteine synthetase. At the same time 20 mM of BSO was added to the drinking water.

### Tissue Glutathione Assay

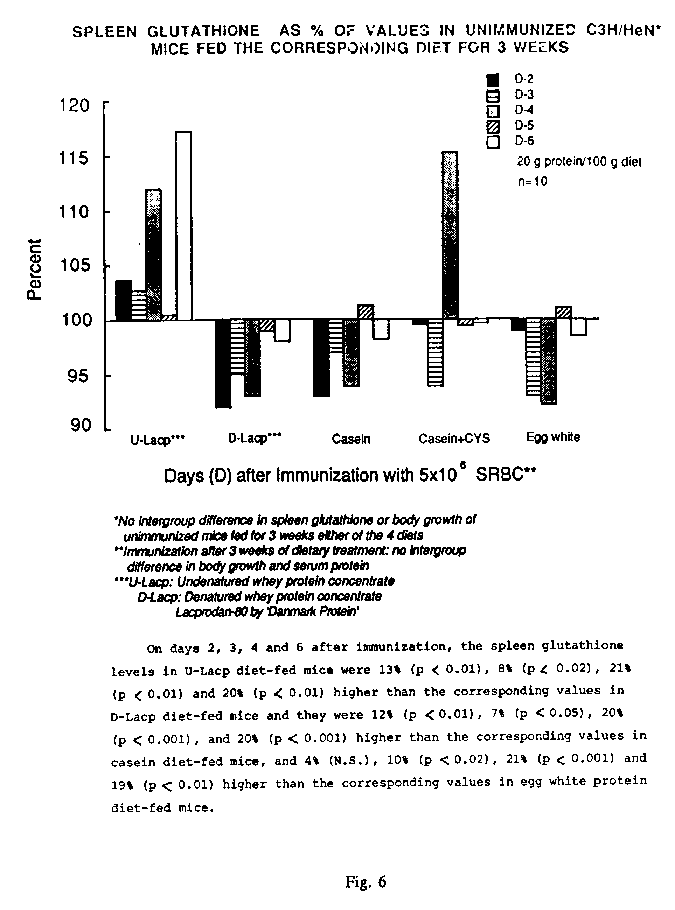

Ninety milligrams of mouse heart or liver were homogenized in 5-sulfosalicylic acid (5% w/v). Homogenates are centrifuged for 5 minutes in a microfuge at 10,000 x g. The assay is carried out using the supernatants on the same day according to the method of Anderson (42). Values are expressed as »mol/g wet tissue (Fig. 6, 8 and 9).

The invention now is described in detail below with reference to drawings in which:
- Fig. 1 and 2: show plaque forming cells per spleen (PFC) on the day showing peak production of PFC following immunization with SRBC,
- Fig. 3: shows the effect of various sources of whey protein concentrate and casein on spleen PFC response,
- Fig. 4: shows the inhibitory effect of heat denaturation on the immunoenhancing property of WPC,
- Fig. 5: shows the enhancement of spleen cell immune response to SRBC following immunization in U-Lacp fed mice,
- Fig. 6: shows spleen glutathione levels in immunized mice expressed as % of values in unimmunized mice fed the corresponding diets,
- Fig. 7: shows the effect of administration of BSO to whey protein diet-fed mice,
- Fig. 8 and 9: show the liver glutathione and heart glutathione content after long term feeding,
- Fig. 10: shows the survival curve of purina, casein and whey protein diet-fed mice,
- Fig. 11: shows the effect of 26 days dietary treatment on PFC response to SRBC.

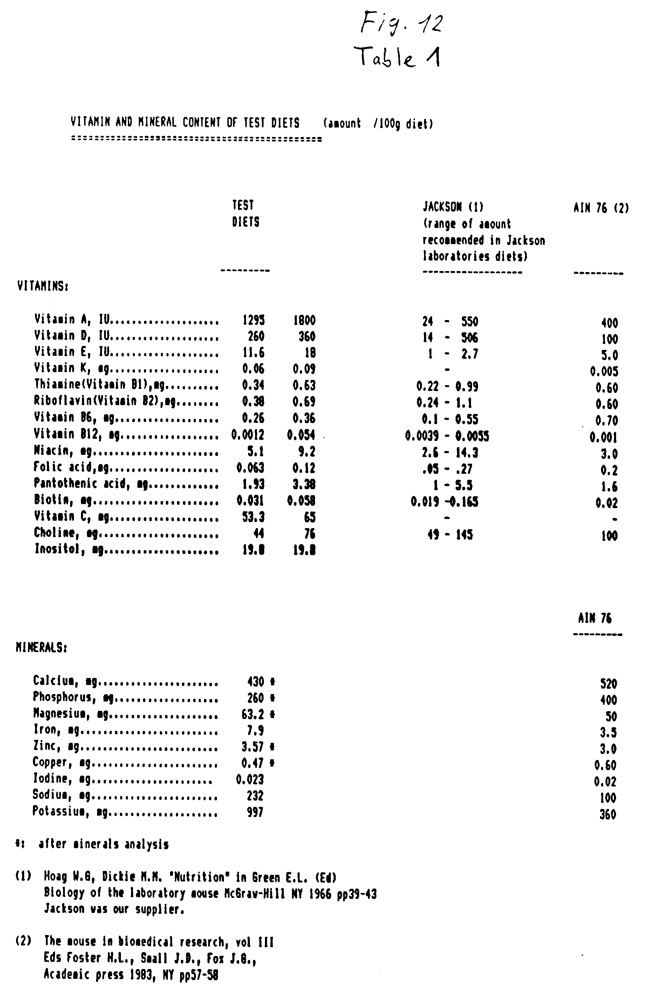

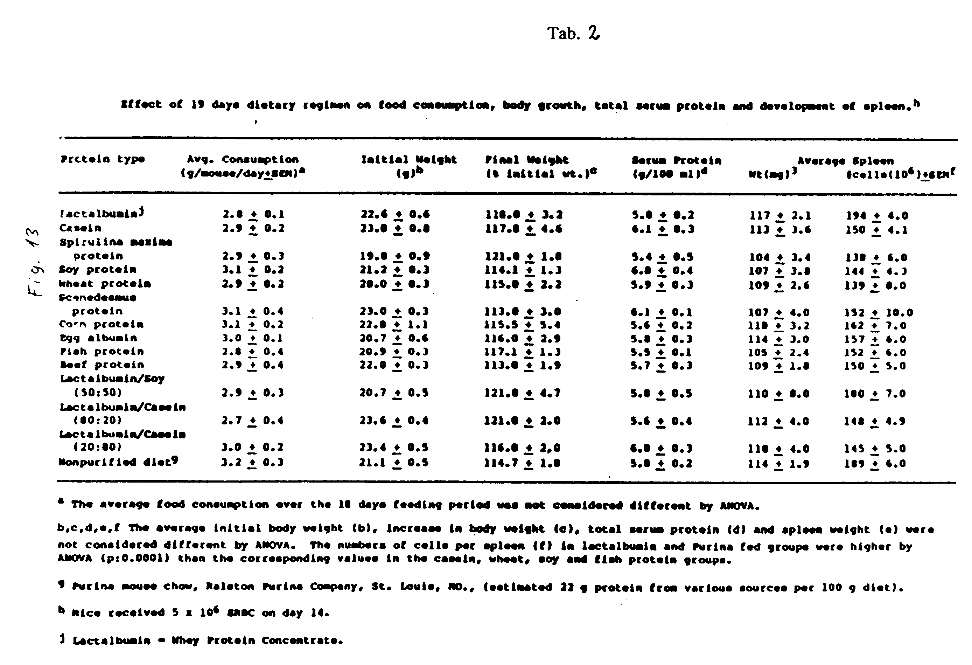

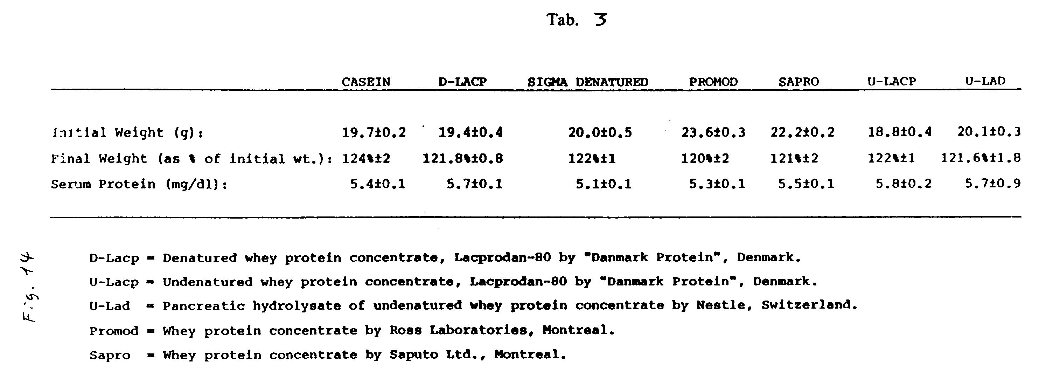

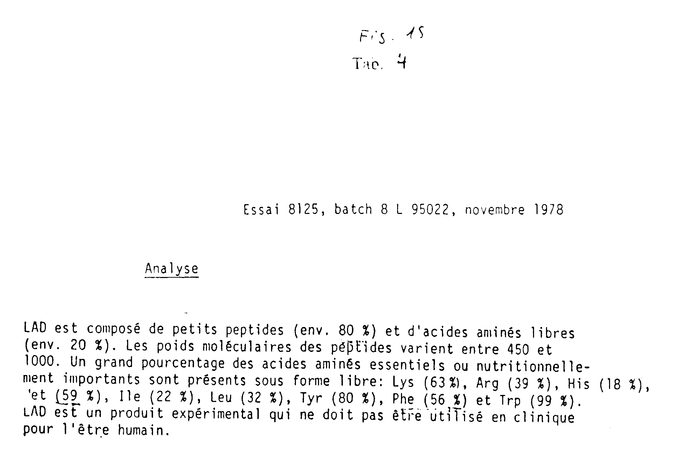

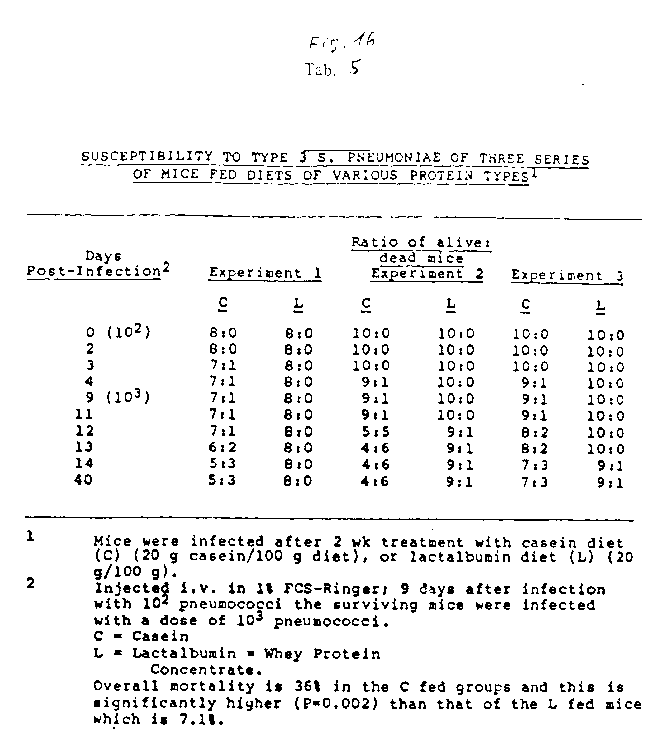

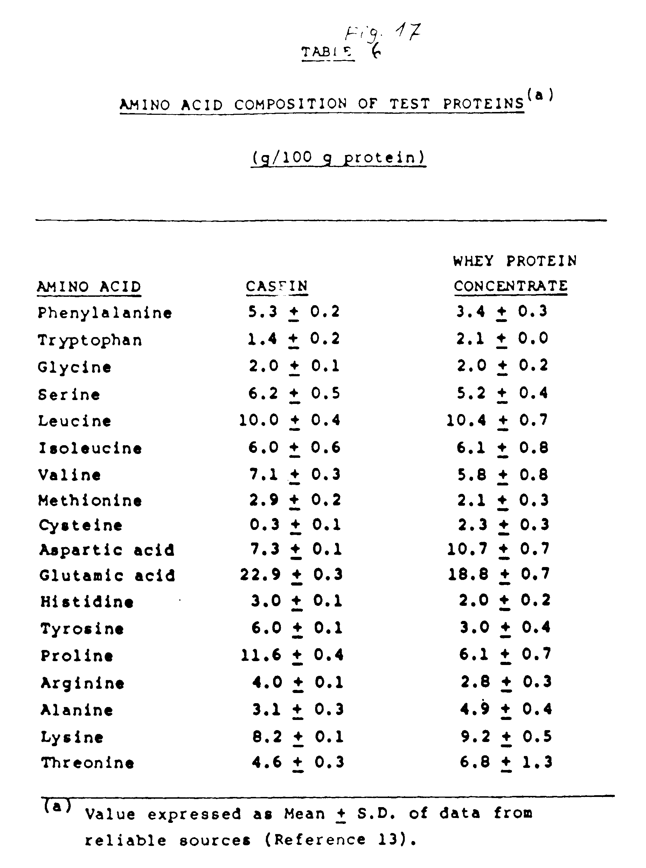

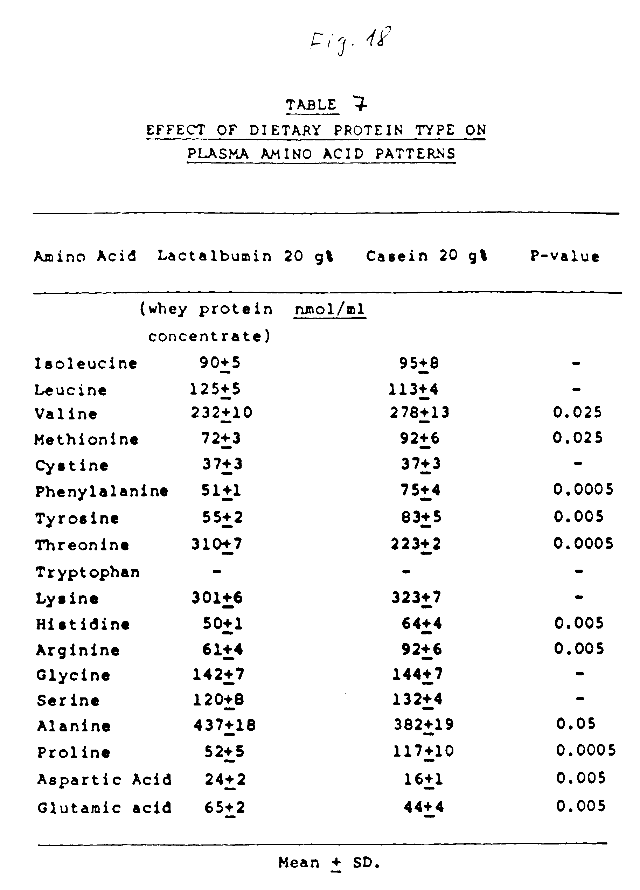

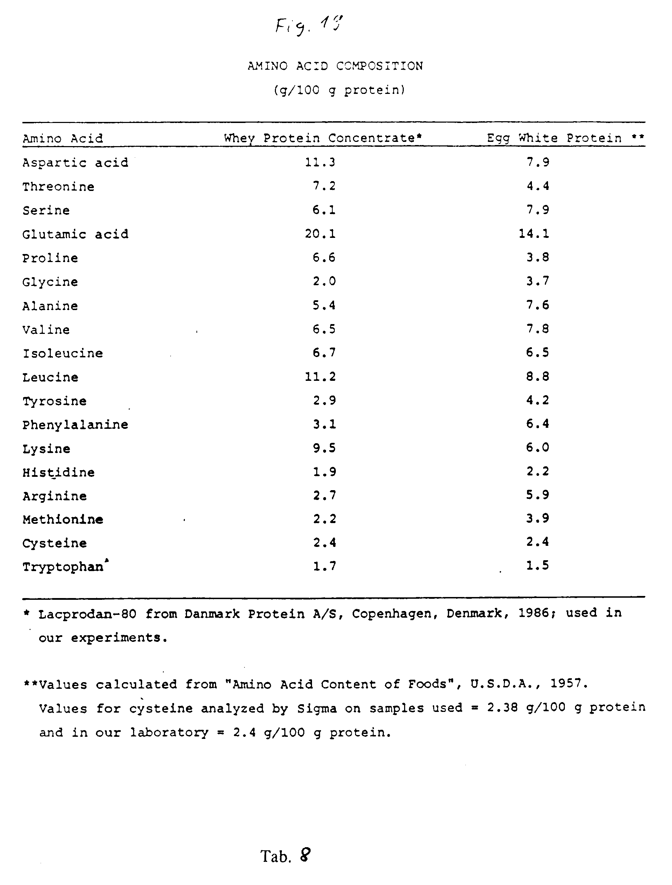

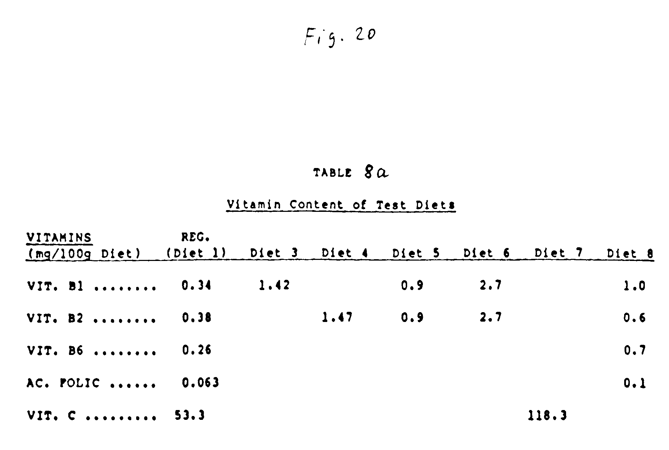

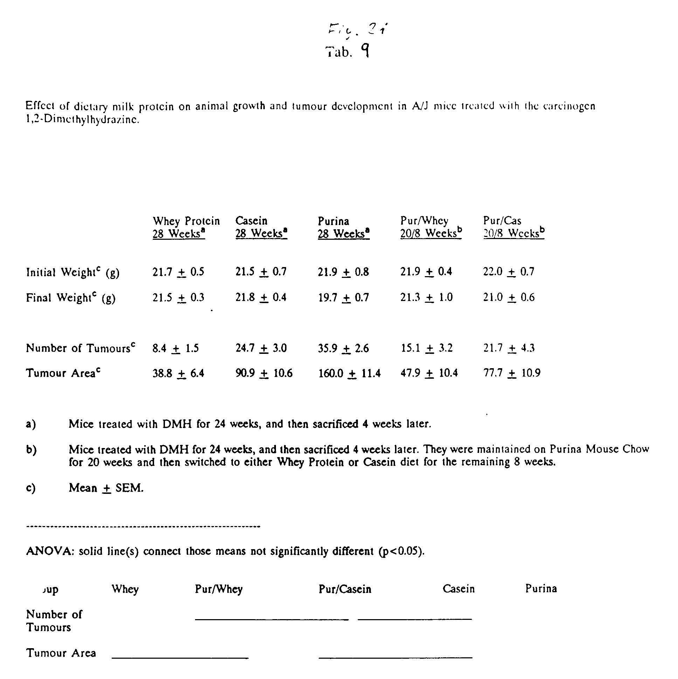

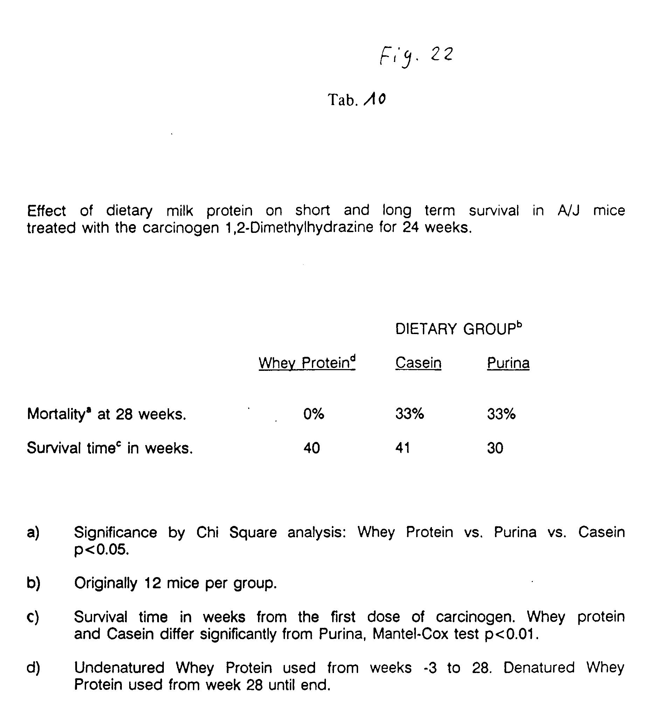

- Fig. 12 - 22: show the tables 1 - 1O
Fig. 1 shows plaque forming cells/spleen (PFC) on the day showing peak production of PFC following immunization with 10⁶ SRBC. Effect of 2 weeks of dietary treatment with 20 g/100 g diet of either whey protein concentrate (L), casein (C), Spirulina maxima protein (Sp), soy protein (S), wheat protein (W), Scenedesmus protein (Sc), corn (CO) protein, egg white protein (E), beef protein (B), fish protein (F), Purina Mouse Chow (P), or 20 g/100 g diet of a mixture containing 50% L and 50% S (L/S), or 80% L and 20% C, or 20% L and 80% C (L/C). Each value represents the mean ± SD.

As indicated in Fig. 1, mice fed the wpc diet for 2 weeks exhibit a plaque forming cell response to sheep red blood cells which is higher than that of mice fed any other protein type or Purina mouse chow. The mean number of plaque forming cells per spleen 5 days after i.v. injection with 5 x 10⁶ sheep red blood cells in the lactalbumin diet-fed mice was 487%, 494%, 736%, 927%, 309%, 284%, 230%, 214%, and 177% of that noted in casein, Spirulina, soy protein, wheat protein, Scenedesmus, corn protein, egg albumin, beef or fish protein diet-fed mice respectively, and 168% of that of Purina-fed mice. These differences are all statistically significant (P = 0.0004). The number of plaque forming cells per spleen in Purina-fed mice was 170% of that in corn protein diet-fed mice (P = 0.0005) and the value of the latter group was 171% of that noted in casein-fed mice (P = 0.0005). No signification difference was observed between fish protein diet-fed, beef protein diet-fed and Purina-fed groups.

The addition of wpc to either soy protein or casein produced a significant increment in the humoral immune response of the host. In a 50:50 mixture with soy protein, wpc induced a 4-fold increment in the immune response in comparison to a purely soy protein diet. In an 80:20 mixture with casein, wpc induced a 3-fold increment and, in a 20:80 mixture with this protein, a 2-fold increase in the immune response was seen in comparison to a purely casein diet.

In conclusion, mice fed a wpc diet for at least 2 weeks exhibit a sustained enhancement of the humoral immune response to sheep red blood cells in comparison to mice fed most of the commercially available edible animal or plant proteins in formula diets of comparable nutritional efficiency. This effect persists as long as dietary treatment is continued (up to 2 months has been tested). It is clear that despite great differences in immune response to SRBC, no difference is seen in food consumption, final weight, and serum proteins among mice fed the various purified proteins at 20 g/100 g diet concentration (see Table 2).

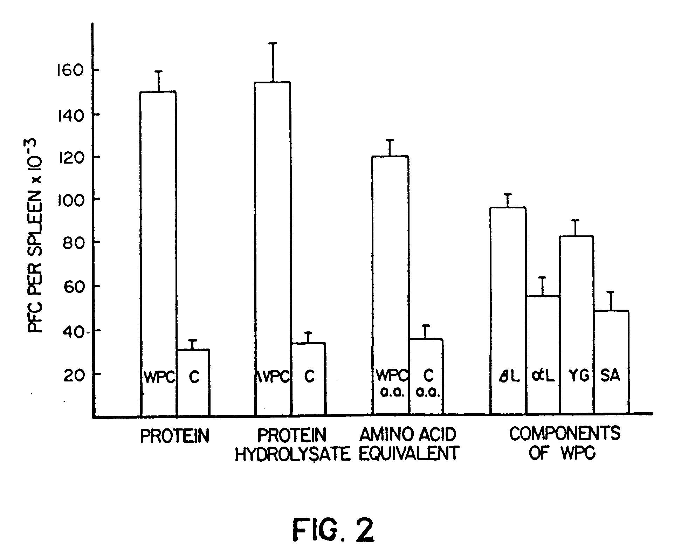

Fig. 2 shows plaque forming cells/spleen (PFC) on the day showing peak production of PFC following immunization with 10⁶ SRBC. Effect of 3 weeks of dietary treatment with 20 g/100 g diet of either whey protein concentrate (WPC), casein (C), whey protein concentrate hydrolysate, casein hydrolysate, beta-lactoglobulin (βL), alpha-lactalbumin (αL), gamma-globulin (γG) or bovine serum albumin (SA). Each value represents the Mean ± SD. When protein hydrolysate was given, the plaque forming cell response in mice fed the whey protein diet was found to be 504% of that noted in the casein diet-fed mice (p = 0.0004) (Fig. 2). When free amino acid mixture was given, the plaque forming cell response in mice fed the whey protein amino acid diet was found to be 332% of that of the casein amino acid diet-fed counterpart (p = 0.0001 (Fig. 2). Our results (Fig. 2) indicate that animals fed diets containing 20 g/100 g diet of any one of the four major components of whey protein (βL, αL, γG, SA) developed a plaque forming cell response to sheep red blood cells inferior to (p = 0.0002) that of mice fed a diet containing 20 g whey protein / 100 g diet.

### Factors responsible for the immunoenhancing effect of whey protein concentrate in diet

### a) Whey protein mixture

The present studies show that the immunoenhancing effect of WPC in comparison to C is maintained, when these two proteins are replaced in formula diets by pancreatic hydrolysate (20% free amino and 80% oligo peptides with MW less than 1000) (Fig. 2). These results also indicate that mice fed diets containing any one of the four major protein components of the WPC mixture developed a PFC response to SRBC inferior to that of mice fed the corresponding whey protein mixture. It can be concluded that the observed immunoenhancing effect of WPC is dependent upon the contribution of all its protein components. For these reasons it can be assumed that this phenomenon is not related to milk protein allergy or some other manifestation of oral immunization.

### (b) Undenatured Conformation of the Whey Protein Concentrate

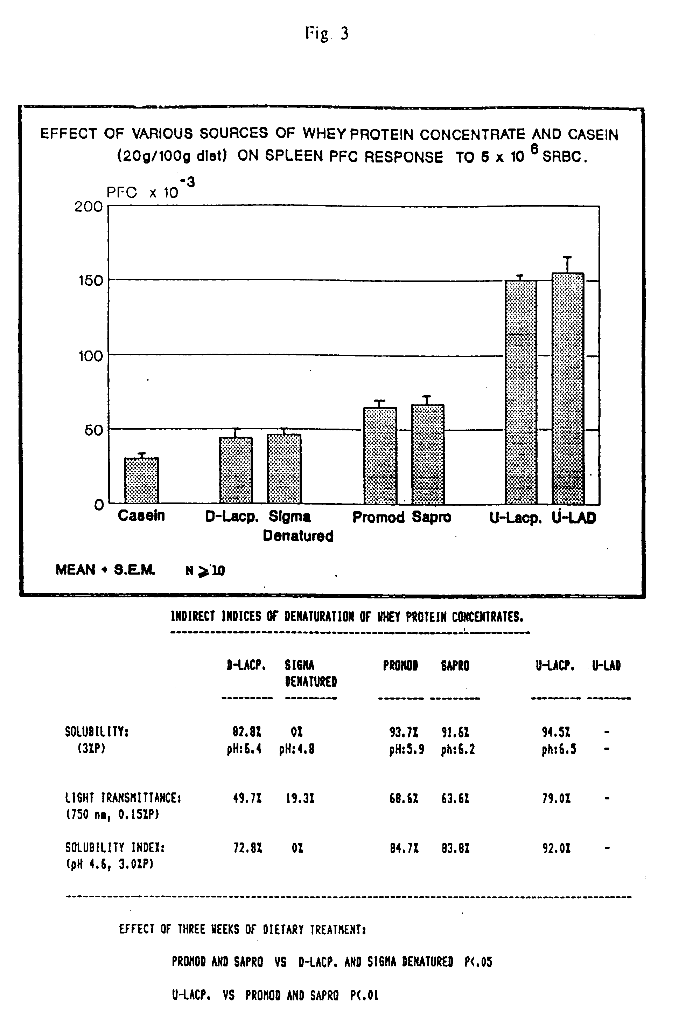

Recent observations have revealed to us that the described biological activity of the whey protein concentrate, already shown to be unrelated to its nutritional quality, is actually dependent on the undenatured conformation of the proteins. This discovery was made accidentally when a batch of whey protein concentrate that was sent to us by the usual supplier failed to exhibit the immunoenhancing effect previously described while exhibiting the same nutritional efficiency. Upon analysis it appeared that this preparation was less soluble and exhibited all the characteristic indirect signs of denaturation (D-Lacp), quite different indeed from the previous samples of undenatured whey protein (U-Lacp) exhibiting strong biological activity. Data on Figure 3 indicate the relationship between the degree of denaturation of whey protein concentrate and the PFC immune response of the host. The related Table 3, further indicates the lack of correlation between nutritional efficiency and denaturation of protein. In the natural state, the milk whey proteins have a definite conformation which, when exposed to heat above a certain critical level, is disrupted. In contrast to caseins, the whey proteins are rapidly denatured by heating. Denaturation of whey proteins causes unfolding of their globular structure to form a random coil configuration. In addition to heating, other processing treatment, e.g. pumping, mixing, aeration, vacuum evaporation and drying further promote protein denaturation.

In the present studies, whey protein concentrate denaturation was evaluated by the following methods: Solubility measurements: After dispersion of a 3% protein solution in distilled water at room temperature and, in some cases, pH adjustment, the solution was stirred and then centrifuged for 20 minutes at 40,000 x g. The protein content of the supernatant was determined by the Lowry method. Percent solubility was computed as the portion of total protein recovered in the supernatant fraction. Light transmittance: The initial 3% protein solution was diluted to 0.15% in distilled H₂O. The light transmittance of blank (distilled H₂O) and sample was measured at 750 nm with the spectrophotometer immediately after mixing.

It is apparent from Figure 3 that a positive relation exists between the undenatured state of whey protein concentrate in the diet and the intensity of the humoral immune response to SRBC.

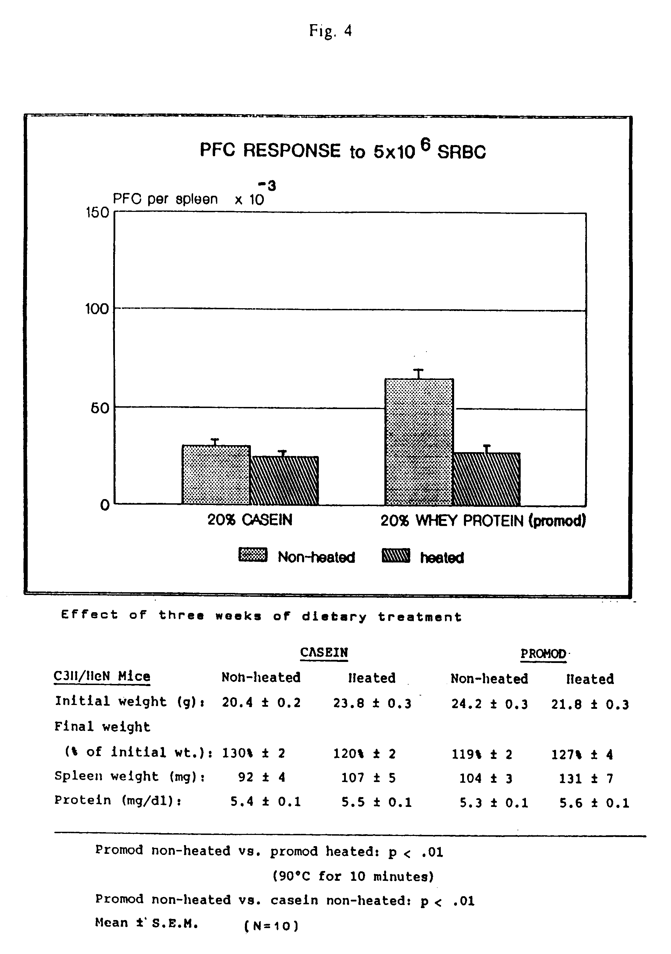

The level of immune response is not related to the nutritional efficiency of the whey protein concentrate but to its undenatured conformation. Hence, the independence of the biological activity (immunoenhancement) from the nutritional aspect of the whey protein concentrate, shown in our previous short term experiments (Figures 1 and 2, Table 2) is confirmed. Further evidence of the inhibitory effect of heat denaturation on the immunoenhancing property of whey protein concentrate was obtained by heating a partially denatured whey protein concentrate (Promod). This procedure produced a significant drop in the immunoenhancing property of the diet without change in its nutritional efficiency (Figure 4).

Preliminary heat treatment of the concentrated whey protein solution will not improve its overall digestibility; hence the whey protein concentrate used in the preparation of the pancreatic hydrolysate LAD was undenatured. The absence of cysteine in the free amino acid fraction of LAD (Table 4) is consistent with the knowledge that pancreatic trypsin does not hydrolyse the disulfide cross-linkage⁽³⁰⁾ characteristic of the native whey protein which is instead split in the process of denaturation.

### DIETARY WHEY PROTEIN AND PNEUMOCOCCAL INFECTION

Because our studies had shown that dietary protein type influences the humoral immune response, we then proceeded to investigate the effect of U-Lacp in diet on the resistance of mice to pneumococcal infection. Pneumococci represent the group of encapsulated high virulence organisms against which the body employs a humoral immune response. C3H/HeJ mice fed a diet containing 20 g U-Lacp/100 g diet showed improved survival after i.v. infection with Streptococcus pneumoniae type 3 as compared to similarly infected mice fed a 20 g C/100 g diet of similar nutritional efficiency (Table 5).

On the basis of our various studies, it was shown that the enhanced resistance of mice fed the whey protein diet to infection with the Streptococcus pneumoniae type 3 was independent of the weight of the animal at the time of infection and the weight gained before infection (animals were fed the diets for 2 weeks prior to infection).

### Mechanism responsible for the immunoenhancing effect of whey protein concentrate in diet

Over the past few years we have attempted to idenfity the changes induced by dietary protein type which might directly or indirectly affect the humoral immune responsiveness. At 20 g/100 g diet concentration, the proteins provide an adequate daily supply of essential amino acids for the growing mice. The only significant effect of protein type was found to be a change in plasma amino acid profile, which essentially conformed to the amino acid composition of the ingested protein, with the notable exception of cysteine (Tables 6 and 7).

It was surprisingly found that, in spite of an 8-fold higher cysteine content in WPC, the plasma level of cysteine in WPC diet-fed mice was not different from that in their C diet-fed counterparts. The fate of the excess cysteine was a matter of interest. Dietary cysteine is a rate limiting substrate for the synthesis of glutathione (GSH) which is necessary for lymphocyte proliferation. GSH is dependent upon the supply of cysteine which is derived from dietary protein. The redox state of the lymphocyte can modulate the intracellular concentration of cyclic GMP, which is known to be intimately involved in lymphocyte proliferation

The present studies have shown that the observed enhancement of the immune response is associated with greater production of splenic glutathione in immunized mice fed whey protein in comparison to mice fed a casein or cysteine enriched casein diet. The efficiency of dietary cysteine in inducing supernormal glutathione levels is greater when it is delivered in the whey protein than as free cysteine (Fig. 6).

### Method to increase tissue glutathione

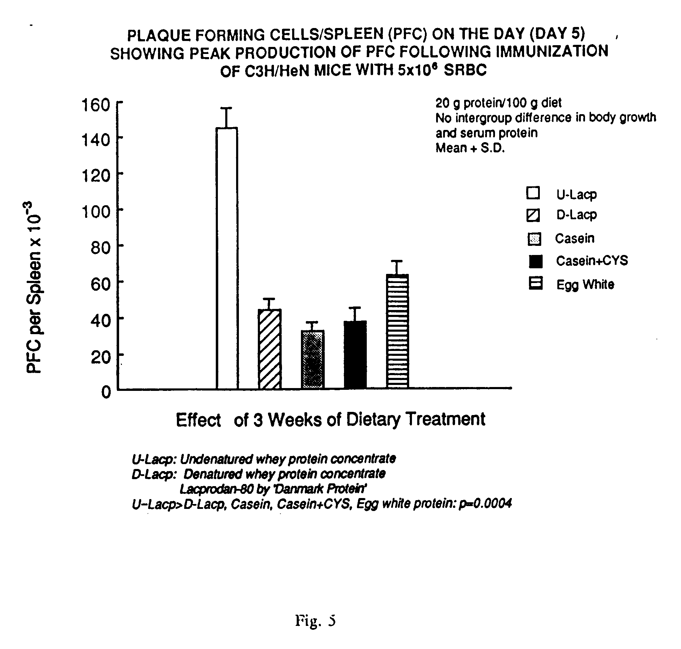

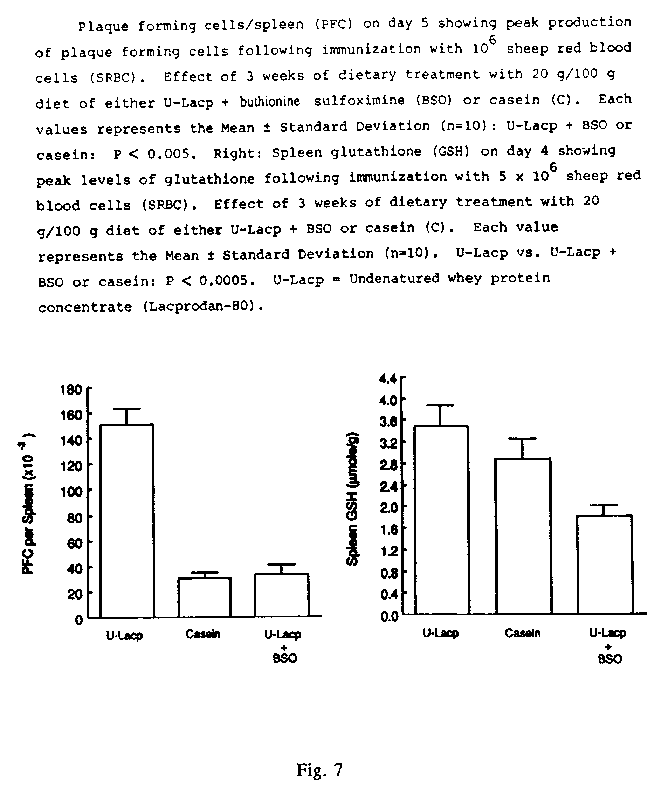

The interaction of dietary protein, GSH and the host immune response was further explored. It was investigated whether a different protein source such as egg white, with the same high level of cysteine as whey protein concentrate (Table 8), had a similar effect in promoting higher GSH tissue content. It was found that an egg white protein diet does not enhance the host immune response above average (Fig. 1). Whereas the static GSH level in spleen was found unaltered by U-Lacp feeding for three weeks, the present studies in young adult C3H mice showed that enhancement of spleen cell immune response to SRBC (Fig. 5) is associated with sustained elevation of splenic GSH during the antigen-driven clonal expansion of the lymphocytes in U-Lacp (undenatured whey protein)-fed mice in comparison to a pattern of decline observed in spleen GSH levels in mice fed either of the nutritionally equivalent D-Lacp (denatured whey protein), casein, cysteine enriched casein, or egg white protein diets (Fig. 6). The latter four groups also exhibited a lower immune response (Fig. 5). Administration of S-(n-butyl) homocyteine sylfoximine, which reduces the splenic glutathione level by half, produces a marked drop in the humoral immune response of whey protein (U-Lacp) diet-fed mice. This is further evidence of the important role of glutathione in the immunoenhancing effect of dietary whey protein (Fig. 7).

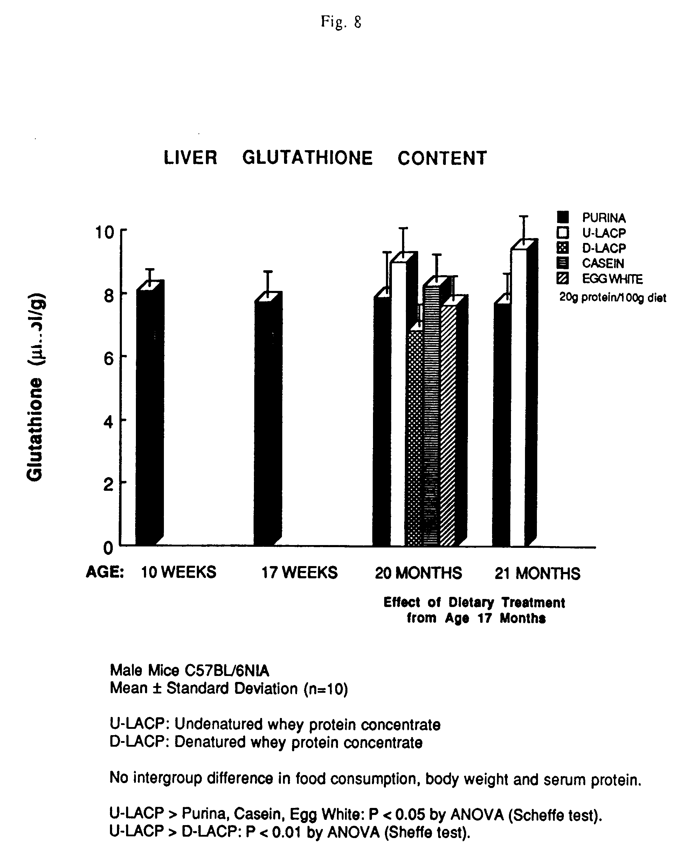

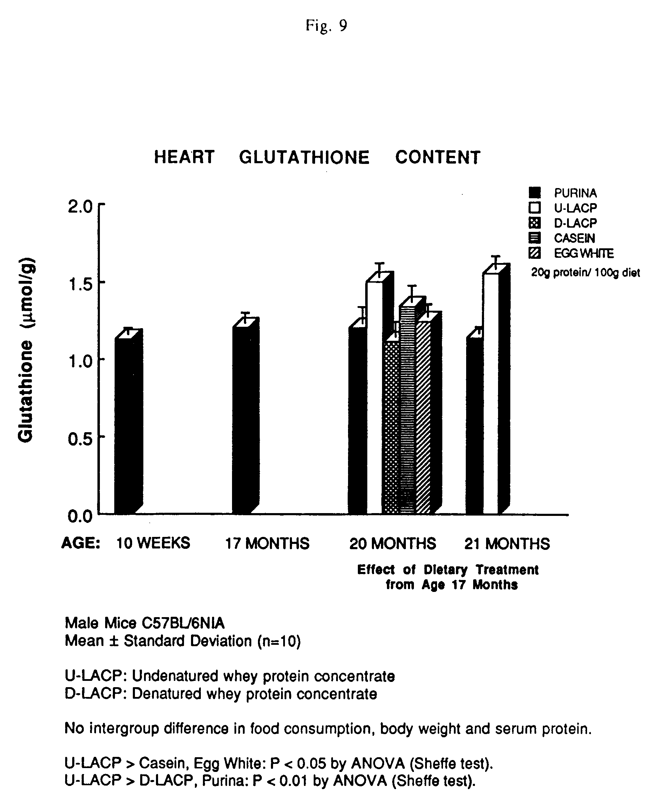

After three months on either diet initiated at age 17 months, GSH content was found to be higher in the liver and heart of U-Lacp (undenatured whey protein) fed mice compared to the D-Lacp (denatured whey protein), casein, egg white protein or Purina diet-fed counterparts (Figures 8 and 9). The GSH values in heart and liver of mice fed Purina laboratory chow was similar at age 10 weeks, 17, 20, 21 months. The U-Lacp diet appears to enhance the GSH content of heart and liver above "normal" values after 3 and 4 months of continuous feeding (Figure 8 and 9).

In conclusion, after three weeks on the U-Lacp diet, spleen GSH content is increased during the antigen driven clonal expansion of the lymphocytes in young adult C3H/HeN mice as compared to a decline in controls fed D-Lacp, casein or egg white protein diets (Figure 6). In old C57BL/6N1A mice, long term feeding of U-Lacp diet results in a moderate but sustained increase in liver and heart GSH levels (Figures 8 and 9). The GSH enhancing activity of WPC is restricted to its undenatured form (U-Lacp). This property is not solely due to the high cysteine content of WPC because another protein source with similar cysteine content (egg white) does not exhibit this biological activity. This property of U-Lacp does not depend specifically on its nutritional efficiency as evaluated by body weight, serum proteins, and food consumption, but appears to depend on the primary, secondary and tertiary structure of the protein in its native form.

Data in Figures 8 and 9 show that the concentration of liver and heart glutathione in control Purina fed mice remains very constant over time. On the other hand a moderate but sustained elevation of tissue GSH was noted in mice fed the nutritionally equivalent whey protein (U-Lacp) diet.

Given the fact that cellular GSH is very tightly regulated, that a 2-fold increase may be maximal, and that the effect of small increments in GSH may be amplified by a variety of GSH-utilizing enzymes (e.g. glutathione peroxidase, glutathione-S-transferase), the reproducible change in GSH concentration observed in animals fed the whey-rich diet is likely to have biological importance. The chronic nature of this augmentation may contribute significantly to this effect.

### A method to inhibit the growth of chemically induced colon cancer

In light of the present findings on the lability of the biological property of whey protein concentrate, it is conceivable that the whey protein fraction of the milk protein mixture, used in the later experiments (57), was partially or totally denatured. In spite of variations in the type of tumor and in the control diets used in all the studies mentioned in prior art, it is apparent that the level of tumor inhibition reported with dairy product feeding is comparable to that which we obtained with a formula diet containing casein as protein source.

The present findings show that in mice fed a casein diet the number and size of DMH induced colon carcinoma were reduced by a factor of 0.3 and 0.4 respectively in comparison to Purina fed controls (Table 9). However, in mice fed the whey protein diet with similar nutritional efficiency the number and size of DMH-induced colon carcinoma were reduced four fold in comparison to the Purina fed controls (Table 9). The superiority of the anti-cancer effect of whey protein in comparison to casein has been reported in our previous study. It is apparent from the above described studies that the antitumor activity of the dairy products is in the protein fraction and more specifically, as the present invention demonstrates, in the whey protein component of milk.

The amount of whey protein concentrate given orally to our mice can be estimated at 0.8 g protein a day, hence about 20,000 times more protein than was injected in GB-A-1495940.

This would make the prior art not comparable to the present claim where a specific protein mixture (whey protein concentrate) was found to exert its anti-cancer effect after long-term oral feeding, digestion and absorption by a physiological route which essentially rules out, particularly in the adult mouse, absorption of antigenic material. Moreover, other foreign proteins such as casein or those various proteins contained in Purina did not exhibit an anti-cancer effect when given orally in similar amounts.

The present studies have consistently shown that the anti-cancer activity of dietary whey protein concentrate, in its undenatured form, correlates directly with the PFC assay employed in our experiments. This immunoenhancing activity of WPC results from the contribution of all its protein components. In the present studies, mice fed the fractions of WPC (6000 - 20,000 mol. wt.) shown in GB-A-1495940 to exert the antileukemic effect by i.p. injection (α-lactalbumin, β-lactoglobulin), developed a PFC response to SRBC significantly less than that of mice fed the whole whey protein concentrate (Fig. 2). Hence, to the extent that the anticancer effect of WPC correlates with the immunoenhancing activity of WPC our claim is not related to the phenomenom reported in GB-A-1495940.

Finally, not only concentration but, because of the different routes of administration, type and metabolic fate of biologically active intermediary products differ in the two studies.

### SURVIVAL STUDIES: THE BIOLOGICAL ACTIVITY IS DEPENDENT ON THE UNDENATURED CONFORMATION OF WPC

### (a) Survival of Old Mice During a Limited Time Period:

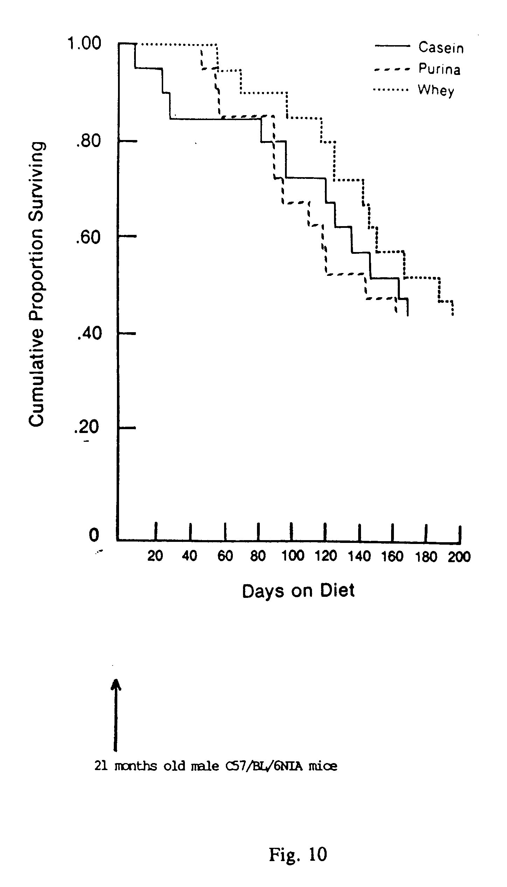

Our study shows that the mean survival time, over a limited observation period of 6-7 months ending when 55% of male C57BL/6N1A mice were dead, is increased by about 30% in mice commenced on the undenatured whey protein (U-Lacp) diet at the onset of senescence (age 21 months) in comparison with "controls" fed the nutritionally equivalent Purina mouse chow. The survival curve of Purina fed mice was very similar to that of casein diet-fed mice (Figure 10). However, in the subsequent four months, mice on undenatured whey protein diet were switched to a denatured whey protein concentrate (D-Lacp) diet. During this period, the time of death of the remaining whey protein diet-fed mice became similar to that of their casein diet or Purina-fed counterparts. Throughout the study repeat bioassays of PFC formation confirmed the correlation between host immunoenhancement and undenatured state of WPC in diet as indicated in Figure 3. In the second part of the study, when the difference between survival curves began to narrow, the immunoenhancing property of WPC was absent although its nutritional quality was preserved (D-Lacp). Throughout the entire study no significant intergroup difference was seen in calorie intake, and body weight. Since longevity is dependent primarily upon the genome of the individual it is unlikely that delayed mortality over a limited period of time would have influenced overall longevity. However, at least in terms of the immunoenhancing effect of the diet, this study could be regarded as a single direction cross-over from test (U-Lacp) to control (D-Lacp) diets, showing that the biological activity of WPC on survival of old mice is dependent upon its undenatured state and correlating directly with the PFC assay used in our study (as illustrated in Figure 3).

### (b) Short and Long Term Survival of Mice with DMH-Induced Colon Cancer:

In DMH treated mice we noticed a difference between mortality by the 28 weeks end point and the survival time to the end of the experiment in relation to dietary protein type. During the first seven months of study, the mice fed undenatured whey protein (U-Lacp) had no death as compared to a 33% mortality observed towards the end of this period in the casein and Purina groups. In the subsequent four months mice on whey protein were fed denatured whey protein (D-Lacp). During this latter period the D-Lacp diet appeared to have no favourable effect on survival in comparison to the casein diet (Table 10). Throughout the study repeat bioassays of spleen PFC were done to document the physiologic effects of the diets on immune function as reported previously and the stability of these effects. The immunoenhancing effect of the U-Lacp diet was consistently confirmed for the first 7 months of the study; however, in the following four months (D-Lacp), the immunoenhancing effect previously observed in mice fed the U-Lacp diet was absent. The values of PFC response in relation to either the U-Lacp diet or the D-Lacp diet were consistent with those presented in Figure 3. This study therefore confirms the hypothesis that the biological activity of WPC on survival of tumor bearing mice is dependent upon its undenatured state correlating directly with the PFC assay used in our study.

### Synergistic role of Vit. B₂, B₁ in the immunoenhancing effect of dietary whey protein concentrate

While whey protein represent an optimal source of cysteine, the rate limiting substrate for the biosyntheses of GSH, Vit. B₂ and B₁ are important elements in the function of the GSH redox cycle.

Glutathione (GSH) status in tissues is maintained mainly in the reduced state (GSH:GSSG, 250), which is achieved by the efficient GSH peroxidase and reductase system coupled to the NADP+/NADPH redox pair. Endogenous toxic H₂O₂ is reduced to H₂O through the oxidation of GSH to GSSG catalyzed by GSH peroxidase. At the expense of cellular NADPH, GSSG is effectively reduced back to GSH by NADPH:GSSG reductase, thus maintaining thiol balance. As a result, GSSG reductase has a great capacity to protect cells against oxygen toxicity from endogenous active oxygen species.

Vit. B₁ (thiamine) is involved in the transketolase reaction of the pentose phosphate shunt yielding NADPH and pentose.

Vit. B₂ (riboflavin): The coenzyme derivatives of riboflavin, flavin mononucleotide (FMN) and flavin adenin dinucleotide (FAD), are synthesized sequentially from riboflavin. Vit. B₂ deficient animals exhibit marked decreases in activities of FMN and FAD-requiring enzymes, such as GSH reductase.

In this sense, it is conceivable that all these water soluble vitamins naturally present in whey, play an essential role for optimal function of the GSH redox cycle particularly when whey protein intake, as shown in our experiments, has produced higher level of GSH synthesis and storage in the tissues.

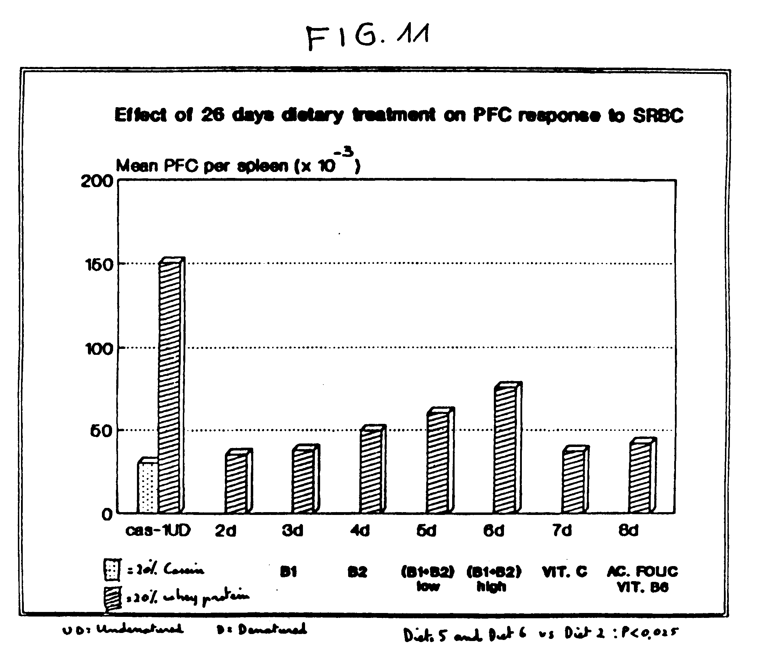

The present studies (Fig. 11) show that dietary levels of Vit. B₁, B₂ slightly above recommended allowance (Table 8a, diets 5, 6) significantly contribute to the immunoenhancing effect of dietary whey protein concentrate. Whey protein, by providing optimal bioavailability of the limiting substrate (cysteine) enhances the synthesis and storage of GSH. On the other hand, higher than normal intakes of Vit. B₁ and B₂ are necessary to maintain the GSH redox cycle at a level higher than normal, thus allowing the development of a better than normal immune response to SRBC. Individually the effect of each of the vitamins in whey protein fed mice is limited; however, their synergistic effect on the immune response of whey protein fed mice is apparent (Fig. 11, diets 5, 6 diet 1). The same vitamins are ineffective on the immune response of casein diet-fed mice. Although all these water-soluble vitamins are present in whey, it is interesting to note that the main natural source of the single most effective vitamin, riboflavin, is whey to which Vit. B₂ given its characteristic color.

In conclusion, dietary intake of Vit. B₁ and particularly B₂ above recommended daily allowance contribute to the development of enhanced immune response in whey protein fed animals: Vitamin B₂ + B₁ appears to produce the strongest effect. When intake of these vitamins is at or slightly below these levels, body growth and animal appearance are normal, but the response to immune challenge is below the maximum potential of whey protein fed mice. The whey protein composition according to the invention comprises in combination said WPC together with vitamins B₁ and B₂ in amount of 1.5 to 2.0 mg B₁ and 1.5 mg to 2.0 mg B₂.

In the stomach, whey is separated from milk by the action of gastric juice. It is conceivable that the transit and absorption of the water-soluble vitamins and proteins of whey occur faster than those of the protein (casein) and vitamin constituents of the milk coagulum (curd). Hence the whey protein and vitamins including the vitamins B₁ and B₂ could enter the systemic circulation at a different rate than that of other milk constituents and express their synergistic effect on the immune system and the GSH redox cycle.

The immunoenhancing and the other specific biological properties of dietary whey protein described in this application, are heat labile and dependant upon the undenatured (native) state of the protein (which can also be affected by vigorous shaking, solvents, extreme ph changes, etc.) and are independent of its nutritional quality which is unaltered by the process of denaturation.

Unlike most other commercially available whey protein which are denatured, the whey protein used in our experiments, produced in Denmark (Lacprodan - 80) is 90% undenatured (U.D. in Fig. 11). This protein displays the greatest tendency to denature under heat thus exposing its free sulfhydryl group. When experiments were done using a batch of w.p.c. received after a long surface transport from Denmark through the U.S. in exceptionally hot and humid weather (summer 1988), the immunoenhancing property of w.p.c. was lost (Fig. 11, 2d-8d). These experiments, while indicating the synergistic role of vit. B₁ and B₂, in the immunoenhancing effect of the diet, also show the negativ effect of a presumably partially denatured whey protein. Previous studies have shown, that the immunoenhancing property of dietary whey protein is probably related to an optimal intracellular transport and availability of the cysteine which is a limiting precursor for glutathione synthesis. It is conceivable that partial denaturation of this protein had brought about the loss of its specific biological property by altering a cysteine and GSH synthesis, without any effect on its nutritional quality.

### Milk processing and whey preparation

Immediately after milking, the milk is cooled to 4°C and kept in a cooling tank for delivery to the cheese factory. The precipitation of the curd is obtained by reducing the pH to about 4.6 with lactic acid initially at 20°C. Following the addition of rennet (normally three ounces / 1000 pounds (=85g/453,6 kg) of milk), the temperature is raised to about 30°C for 20 minutes to promote expulsion of whey from the curd, allowing the agitation in the vat to resolve at low speed.

When sufficient quantity of whey is obtained, the product remaining in the vat is pasteurized in the standard fashion to obtain reduction of bacteria and agitated at high speed for cheese production. The whey is then irradiated with a source of gamma-irradiation. The radiation dose will vary from 5 to 15 kGy according to bacteria content of the whey, to reach equivalent antibacterial effect of standard pasteurization with minimal protein denaturation (measured by changes in soluble protein, i.e. protein concentration in whey before and after treatment).

The difference between our method and the standard method is that the fraction of whey to be used for subsequent production of whey protein concentrate is not heated and the material from which it is derived is slowly agitated to minimize protein denaturation. The prevention of denaturation by maintaining high solubility avoids coprecipitation of whey proteins with the caseins, thus increasing the protein content of whey. The whey is then cooled to 6°C.

### Whey protein concentration and isolation

For the production of undenatured whey protein concentrate, the whey is separated and concentrated through ultra-filtration, which allows for selective separation of protein from lactose, salts and water under mild conditions in which a pressurized solution flows over a porous membrane. The membrane allows the passage of only relatively small molecules.

To prevent excessive microbial growth during residence time and protein denaturation, the plant is operated below 10°C most of the time. A thin layer membrane of polymeric material (polysulphone) with a cut off value of approximately 10,000 is used, so that protein components of MW 15,000 are retained. To speed up filtration, the liquid is fed on the membrane at a pressure of 5 bar (kg/cm²).

A frame-type module is constructed to hold a large number of these membranes. The production line consists of 18 such modules. In the last 10 modules, demineralized water is added and then removed through the membranes carrying lactose and minerals with it. To maintain velocities adequate to minimize concentration polarization and fouling, recirculation pumps are used in each stage.

A final protein concentrate with 80% protein (undenatured) in dry matter can be thus achieved.

### Relevance of experimental data on whey protein concentrate to humans

The immunoenhancing activity of undenatured whey protein concentrate was described in five unrelated strain of male mice.

The anticancer effect of whey protein concentrate (w.p.c.) was noted in a different strain of female mice.

Birt et al (3, 4) showed increased longevity in w.p.c. fed hamsters of both sexes.

The biological activity of w.p.c. appears then to be independent of specific genetic or hormonal influences. Moreover it is generally accepted that if a biological phenomenon of this type occurs in two different species, it should apply to other species including man.

The type of colon cancer against which w.p.c. was found to be effective is similar to the human colon cancer in hystological appearance and in its response to chemotherapy.

Premature human babies, unlike full term infants, require whey predominant formulas to prevent metabolic acidosis and to survive.

Perhaps more importantly human milk has by far the highest whey protein / casein ratio compared to any other mammal. Presumably nature has prepared humans through the only obligatory form of nutrition, to handle undenatured whey proteins for their best metabolic advantage. In fact one would anticipate that the favourable biological activity of w.p.c. observed in rodents might be more pronounced in the human host.

### References

1. BARICELLI, G.C. De seri facultatibus, et usu, opusculum secundum. Scorriggium, Publ. Naples, Italy, p. 105-147, 1603.
2. HOFFMAN, K.F. Zur Geschichte der Molkenkuren, insbesondere im 17., 18. und 19. Jahrhundert, Med. Monatschr. 15: 411-416, 1961.
3. BIRT, D.F., BAKER, P.Y., HRUZA, D.S. Nutritional evaluation of three dietary levels of lactalbumin throughout the lifespan of two generations of syrian hamsters, J. Nutrit. 112: 2151-2160, 1982.
4. BIRT, D.F., SCHULDT, G.H., SALMASI, S. Survival of hamsters fed graded levels of two protein sources. Lab.Animal Sci. 32: 363-366, 1982.
5. BOUNOUS, G., STEVENSON, M.M., KONGSHAVN, P.A.L. Influence of dietary lactalbumin hydrolysate on the immune system of mice and resistance to Salmonellosis. J. Infect. Dis. 144: 281, 1981.
6. BOUNOUS, G., KONGSHAVN, P.A.L. Influence of dietary proteins on the immune system of mice. J. Nutr. 112: 1747-1755, 1982.
7. BOUNOUS, G., LETOURNEAU, L., KONGSHAVN, P.A.L. Influence of dietary protein type on the immune system of mice. J. Nutr. 113: 1415-1421, 1983.
8. BOUNOUS, G., KONGSHAVN, P.A.L. Differential effect of dietary protein type on the B-cell and T-cell immune responses in mice, J. Nutr. 115: 1403-1408, 1985.
9. BOUNOUS, G., SHENOUDA, N., KONGSHAVN, P.A.L., OSMOND, D.G. Mechanism of altered B-cell response induced by changes in dietary protein type in mice. J. Nutr. 115: 1409-1417, 1985.
10. EIGEL, W.N., BUTLER, J.E., ERNSTROM, C.A. ET AL. Nomenclature of proteins of cow's milk: Fifth revision. J. Dairy Sci. 67: 1599-1631, 1984.
11. JOHN, A.M., BELL, J.M. Amino acid requirements of the growing mouse. J. Nutr. 106: 1361-1367, 1976.
12. The mouse in biochemical research, Volume III, H.L. Foster, J.D. Small, J.G. Fox, editors, Academic Press, p. 58, 1983.
13. HOAG, W.G., DICKIE, M.M. Nutrition in; Biology of the laboratory mouse, E.L. Green, editor, McGraw-Hill, New York, pp. 39-43, 1966.
14. CANTANI, A. Latte e siero di latte, in; Materia medica e terapeutica, volume I, Vallardi, F. (Publ.), Milano, Italy, p. 385, 1869.
15. SCHAEDLER, R.W., DUBOS, R.J. Effect of dietary proteins and amino acids on the susceptibility of mice to bacterial infections. J. Exp. Med. 110: 921-934, 1959.
16. JANAS, L.M., PICCIANO, M.F., HATCH, T.F. Indices of protein metabolism in term infants fed human milk, whey-predominant formula, or cow's milk formula. Pediatrics 75: 775-784, 1985.
17. DARLING, P., LEPAGE, G., TREMBLAY, P., COLLET, S., KIEN, L.C., ROY, C.G. Protein quality and quantity in preterm infants receiving the same energy intake. Am. J. Dis. Child. 139: 186-190, 1985.
18. SHENAI, J.P., DAME, M.C., CHURELLA, H.R., REYNOLDS, J.W., BABSON, S.G. Nutritional balance studies in very-low-birth-weight infants: Role of whey formula. J. Ped. Gastroent. and Nutr. 5: 428-433, 1986.
19. RAIHA, N.C.R., HEINONEN, K., RASSIN, D.K., GAULL, G.E. Milk protein quantity and quality in low-birth-weight infants: I. Metabolic responses and effects on growth. Pediatrics 57: 659-674, 1976.
20. MOSKOWITZ, S.R., PEREIRA, G., SPITZER, A., HEAF, L., AMSEL, J., WATKINS, J.B. Prealbumin as a biochemical marker of nutritional adequacy in premature infants. J. Pediatr. 102: 749-753, 1983.
21. STARLING, E.H. On the absorption of fluids from the connective tissue spaces. J. Physiol. (London) 19: 312-326, 1896.
22. SCHWARTZ, D.B., DARROW, A.K. Hypoalbuminemia-induced diarrhea in the enterally alimented patients. Nutr. in Clin. Practice 3: 235-237, 1988.
23. BOUNOUS. G. Elemental diets in the prophylaxis and therapy for intestinal lesions: An update. Surgery 105: 571-575, 1989.
24. SHERMAN, P., FORSTNER, J., ROOMI, N., KHATRI, I., FORSTNER, G. Mucin depletion in the intestine of malnourished rats. Am. J. Physiol. 248: G418-G423, 1985.
25. BOUNOUS, G., MCARDLE, A.H., HODGES, D.M., GURD, F.N. Biosynthesis of intestinal mucin in shock: Relationship to tryptic hemorrhagic enterities and permeability to curare. Ann. Surg. 164: 13-22, 1966.
26. BOUNOUS, G. Acute necrosis of the intestinal mucosa: Progress article. Gastroenterology 82: 1457-67, 1982.
27. SHAHANI, K.M., AYEBO, A.D. Role of dietary lactobacilli in gastrointestinal microecology. Amer. J. Clin. Nutr. 33: 2448, 1980.
28. PERDIGON, G., deMACIAS, M.E.N., ALVAREZ, S., ET AL. Systemic augmentation of the immune response in mice by feeding fermented milks with lactobacillus casei and lactobacillus acidophilus. Immunology 63: 17-23, 1988.
29. DE WIT, J.N. New approach to the functional characterization of whey proteins for use in food products, in; Milk proteins 1984, T.E. Galesloot, B.J. Tinbergen, Pudoc. Wageningen, p. 183-195, 1985.
30. LEHNINGER, A.L. Principles of biochemistry. Worth Publ. Inc. p. 688, 1982.
31. ORRENIUS, S., THOR, H., BELLOMO, G., MOLDEUS, P. Glutathione and tissue toxicity. 9th International Congress of Pharmacology, London, July 30th - August 3rd, 1984. W. Patton, J. Mitchell (eds.), McMillan Press (Publ.) P. 57-66, 1984.
32. NOELLE, R.J., LAWRENCE, D.A. Determination of glutathione in lymphocytes and possible association of redox state and proliferative capacity of lymphocytes. Biochem. J. 198: 571-579, 1981.
33. MEGAW, J.M. Glutathione and ocular photobiology. Current Eye Res. 3: 83-87, 1984.
34. AMES, B.N. Food constituents as a source of mutagens, carcinogens, and anticarcinogens. Prog. Clin. Biol. Res. 206: 3-32, 1986.
35. BLUMBERG, J.B., MEYDANI, S.N. Role of dietary antioxidants, in aging, In; Nutrition and Aging, M.L. Hutchinson, H.N. Munro, (eds.), Academic Press, New York, p. 85-97, 1986.
36. HAZELTON, G.A., LANG, C.A. Glutathione contents of tissues in the aging mouse, Biochem. J. 188: 25-30, 1980.
37. HARDING, J.J. Free and protein-bound glutathione in normal and cataractous human lenses. Biochem. J. 117: 957-960, 1970.
38. TATEISHI, N., HIGASHI, T., SHINYA, S., NARUSE, A., SAKAMOTO, Y. Studies on the regulation of glutathione level in rat liver. J. Biochem. 75: 93-103, 1974.
39. ESTRELA, J.M. SAEZ, G.T., SUCH, L., VINA, J. The effect of cysteine and N-acetylcysteine on rat liver glutathione (GSH). Biochem. Pharmacol. 32: 3483-3485, 1983.
40. WILLIAMSON, J.M., MEISTER, A. Stimulation of hepatic glutathione formation by administration of L-2-oxo-thiazolidine-4-carboxylate, a 5-oxo-L-prolinase substrate. Proc. Natl. Acad. Sci. U.S.A. 78: 936-939, 1981.
41. ANDERSON, M.E., MEISTER, A. Transport and direct utilization of γ-glutamylcyst(e)ine for glutathione synthesis. Proc. Natl. Acad. Sci. U.S.A. 80: 707-711, 1983.
42. ANDERSON, M.E. Tissue glutathione: In; Handbook of methods for oxygen radical research. C.R.C. Press, 317-329, 1985.
43. RICHIE, J.P. MILLS, B.J. LANG, C.A. Correction of a glutathione defiency in the aging mosquito increases its longevity. Proc. Soc. Exp. Biol. Med. 184: 113-117, 1987.
44. FERNANDEZ-CHECA, J.C., OOKHTENS, M., KAPLOWITZ. Effects of chronic ethanol feeding on rat hepatocyte glutathione. J. Clin. Invest. 83: 1247-1252, 1989.
45. LAUTERBURG, B.H., MITCHELL, J.R. Therapeutic doses of acetaminophen stimulate the turnover of cysteine and glutathione in man. J. Hepatol. 4: 206-211, 1987.
46. ROUS, P. The influence of diet on transplanted and spontaneous mouse tumors. J. Exper. Med. 20: 433-451, 1914.
47. WHITE, R.F., BELKIN, M. Source of tumor proteins. Effect of a low-nitrogen diet on the establishment and growth of a transplanted tumor. J. Natl. Cancer Inst. 5: 261-263, 1945.
48. NEWBERNE, P.M., CONNER, M.W. Dietary modifiers of cancer, in; Nutrition, Growth and Cancer, pp. 105-129, Alan R. Riss, Inc., 1988.
49. HAWRYLEWICZ, E.J. HUANG, H.H., LIU, J.M. Dietary protein, enhancement of N-nitrosomethylurea-induced mammary carcinogenesis, and their effect on hormone regulation in rats. Cancer Res. 46: 4395-4399, 1986.
50. VISEK, W.J. Dietary protein and experimental carcinogenesis. Adv. Exp. Biol. 206: 163-186, 1986.
51. JACQUET, J., HUYNH, C.H., SAINT, S. Nutrition et cancer expérimental: cas du lait. C.R. Hebd. Seanc. Acad. Agric. de France. 54: 112-120, 1968.
52. HIRAYAMA, T. An epidemiological study on the effect of diet, especially of milk on the incidence of stomach cancer. Abstr. 9th Int. Cancer Congress, Tokyo, Japan 713, 1966.
53. IARC International Microecology Group. Dietary fibre, transit-time, fecal bacteria, steroids, and colon cancer in two Scandinavian populations. Lancet II 207-211, 1977.
54. REDDY, G.V., FRIEND, B.A., SHAHANI, K.M., AND FARMER, R.E. Antitumor activity of yogurt components. J. Food Protect. 46: 8-11, 1983.
55. NUTTER, R.L., GRIDLEY, D.S., KETTERING, J.D., ANDRES, M.L., APRECIO, R.M., SLATER, J.M. Modification of a transplantable colon tumor and immune responses in mice fed different sources of protein, fat and carbohydrate. Cancer Letters 18: 49-62, 1983.
56. GRIDLEY, D.S., KETTERING, J.D., GARAZA, C.D., ANDRES, M.L., SLATER, J.M., NUTTER, R.L. Modification of herpes 2-transformed cell-induced tumors in mice fed different sources of protein, fat and carbohydrate. Cancer Letters 17: 161-173, 1982.
57. NUTTER, R.L., GRIDLEY, D.S., KETTERING, J.D., GOUDE, A.G., SLATER, J.M. BALB/c mice fed milk or beef protein: Differences in response to 1,2-dimethylhydrazine carcinogenesis. J.N.C.I. 71: 867-874, 1983.
58. TSURU, S., SHINOMIYA, N., TANIGUCHI, M., SHIMAZAKI, H., TANIGAWA, K., AND NOMOTO, K. Inhibition of tumor growth by dairy products. J. Clin. Lab. Immunol. 25: 177-183, 1988.
59. ENKER, W.E., JACOBITZ, J.L. Experimental carcinogenesis of the colon induced by 1,2-dimethylhydrazine-di HCl: Value as a model of human disease. J. Surg. Res. 21: 291-299, 1976.
60. CORBETT, T.H., GRISWOLD, D.P., ROBERTS, G.J. ET AL. Evaluation of a single agent and combination of chemotherapeutic agents in mouse colon carcinogenesis. Cancer 40: 2650-2680, 1977.
61. WALSTRA, P., JENNES. Dairy chemistry and physics. Wiley J. Nitork. (ed.), p. 106, 1984.
62. SWAISGOOD, M.E. Characteristics of edible fluids of animal origin: Milk, in; Food Chemistry, O.R. Fennema, (ed.), Marcel Dekker, p. 796, 1985.
63. KIRKPATRICK, K., WALKER, N.J. Casein and caseinates: Manufacture and utilization in; Milk proteins "84", pp. 196-205, T.E. Galesloot, B.J. Tinbergen, (eds.), Pudoc Wageningen, Publishers, 1985.
64. IYNGRARAN, N., YADAV, M. Food allergy, in; Immunopathology of the small intestine, M.N. Marsh (ed.), Wiley J., p. 418, 1987.
65. AMOURIC, M., MARVALDI, J., PICHON, J., BELLOT, F., FIGARELLA, C. Effect of lactoferrin on the growth of a human colon adenocarinoma cell line. Comparison with transferrin. In Vitro 20: 543-548, 1984.
66. GURR, M.I. Review of the progress of dairy science: Human and artificial milks for infant feeding. J. Dairy Res. 48: 519-554, 1981.
67. CUNNINGHAM, A., SZENBERG, A. "Further improvements in the plaque technique for detecting single antibody-forming cells", Prumunology 14/599-600, 1968.

## Claims

1. Method of producing a biologically active whey protein composition comprising a suitable concentration of undenatured whey protein concentrate comprising the following steps:
a) immediately after milking, cooling the milk to a temperature in the range of 2°C to 10°C, especially to 4°C, and removing dirt components,
b) after another cleaning of the milk, precipitation of the curd by reducing the pH to about 4.6 with lactic acid, initially at 20°C,
c) addition of rennet and raising the temperature to about 30°C for 20 minutes to promote expulsion of whey from the curds, allowing the agitation in a vat to resolve at low speed,
d) irradiation and separation of the whey, and
e) a first ultrafiltration of the whey using a membrane having a molecular weight cut off of substantially 10,000 or less,
characterized in that the fraction of whey to he used for subsequent product of whey protein concentrate is maintained at a sufficiently low temperature throughout the process to minimize protein denaturization and the material from which it is derived is slowly agitated to maintain high solvency to avoid co-precipitation of protein with the curd in step c) and to minimize protein denaturation and that said ultrafiltration is carried out in a production line comprising up to 20 frame-type modules holding a large number of said membranes achieving a final undenatured protein concentrate in dry matter, wherein the ultrafiltration is carried out at a temperature in the range of 4°C to 20°C, especially at 4°C.

2. Method according to claim 1 in which the method is carried out mainly at a temperature below 10°C.

3. Method of producing an undenatured whey protein concentrate according to claim 1, characterized in that the whey protein concentrate is free of lactose, salts and water afar ultrafiltration.

4. Whey protein concentrate in dry form obtainable according to claims 1 to 3 for use as a medicine, wherein the whey protein concentrate has immunoenhancing properties being heat-labile and insensitive to pancreatic digestion, and wherein the immunoenhancing properties depend upon the undenatured state of the proteins contained in the whey.

5. Whey protein composition according to claim 4, wherein the whey protein concentrate is one selected from the group consisting of bovine, and/or goat, and/or sheep, and/or human whey protein concentrate.

6. Whey protein composition according to claims 4 and 5, wherein the whey protein concentrate comprises a whey protein isolate mixture in which the major proteins are β- lactoglobulin, α-lactalbumin, immunoglobulin and serum albumin.

7. Whey protein composition according to claims 4 to 6, wherein the whey protein concentrate is for administration of a therapeutical or prophylactical amount of 18 - 28 g whey protein/100 g diet, especially 20 g whey protein/100 g diet.

8. Whey protein contentrate according to claims 4 to 7, wherein the biological activity of the whey protein concentrate is furthermore dependent on the amino acid and small peptide pattern resulting from the contribution of the enhanced fraction and/or fractions.

9. Whey protein composition according to any one of the preceding claims, comprising combination of said whey protein concentrate together with Vitamins B₁ and B₂ in amounts for administration in excess of minimum daily requirements.

10. Whey protein composition according to claim 9, wherein the vitamin amounts are 1.5 - 2.0 mg B₁ and 1.5 - 2.0 B₂/100 g diet.

11. Use of the whey protein compositions according to any one of claims 4 to 10 in a suitable concentration for the manufacture of a substitute for milk or milk protein.

12. Use of the whey protein compositions according to any one of claims 4 to 10 for the manufacture of a food suitable for avoiding the tendency of lactose malabsorption.

13. Use of the whey protein compositions according to any one of claims 4 to 10 for the manufacture of substitutes or complements for dairy products for human or animal alimentation.

14. Use of the whey protein compositions according to any one of claims 4 to 10 for the manufacture of food or food supplement adopted for specific nutrition requirements.

15. Use of the whey protein compositions according to claim 4 for the manufacture of a product for therapeutic objective through specific dietary intervention.

16. Use of the whey protein compositions according to claims 4 to 10 in combination with a neutral vehicle for oral feeding for the manufacture of a diet food having enhanced biological activity.

17. Use of the whey protein compositions according to claims 4 to 10 in combination with a neutral vehicle suitable for oral feeding for the manufacture of an intensive care food having enhanced biological activity.

18. Use of the whey protein compositions according to claims 4 to 10 in combination with a neutral carrier suitable for oral feeding for the manufacture of a pharmaceutical composition having enhanced biological activity.

## Patentansprüche

1. Verfahren zur Herstellung einer biologisch aktiven Molkeprotein-Zusammensetzung, die eine geeignete Konzentration an undenaturiertem Proteinkonzentrat enthält, umfassend die folgenden Stufen:
a) unmittelbar nach dem Melken erfolgendes Abkühlen der Milch auf eine Temperatur im Bereich von 2°C bis 10°C, insbesondere auf 4°C, und Entfernen von Schmutzkomponenten,
b) im Anschluß an ein weiteres Reinigen der Milch erfolgendes Ausfällen von Quark durch Herabsetzung des pH-Werts auf etwa 4,6 mittels Milchsäure bei anfänglich 20°C,
c) Zugeben von Lab und Erhöhen der Temperatur auf etwa 30°C während 20 Minuten zum Fördern der Abtrennung von Molke aus dem Quark unter evtl. Rühren bei geringer Geschwindigkeit in einem Bottich,
d) Bestrahlen und Abtrennen der Molke und
e) erstes Ultrafiltrieren der Molke unter Verwendung einer Membran mit einem Molekulargewichtsschnitt von im wesentlichen 10.000 oder weniger,
**dadurch gekennzeichnet,**
daß die für das anschließende Molkeproteinkonzentrat zu verwendende Molkefraktion während des Verfahrens zwecks Herabsetzung einer Proteindenaturierung bei einer hinreichend niedrigen Temperatur gehalten und das dafür maßgebliche Material zur Vermeidung von einer Mitausfällung von Molke mit Quark in der Stufe c) auch einer Proteindenaturierung schwach gerührt wird, und daß die Ultrafiltration in einer Fertigungsstraße, die bis zu 20 rahmenartige Einheiten mit einer großen Anzahl der besagten Membranen aufweist, unter abschließendem Erhalt von einem undenaturierten Proteinkonzentrat in trockener Form erfolgt sowie diese Ultrafiltration bei einer Temperatur im Bereich von 4°C bis 20°C, insbesondere 4°C durchgeführt wird.

2. Verfahren gemäß Anspruch 1,
**dadurch gekennzeichnet,**
daß es im wesentlichen bei einer Temperatur unterhalb von 10°C durchgeführt wird.

3. Verfahren zur Herstellung eines undenaturierten Molkeproteinkonzentrats gemäß Anspruch 1,
**dadurch gekennzeichnet,**
daß das Molkeproteinkonzentrat nach der Ultrafiltration frei von Lactose, Salzen und Wasser ist.

4. Molkeproteinkonzentrat in trockener Form, erhältlich gemäß den Ansprüchen 1 bis 3 und zur Verwendung als Medizin vorgesehen,
**dadurch gekennzeichnet,**
daß es immunitätserhöhende Eigenschaften aufweist, hitzelabil und unempfindlich gegen pankreatische Digestion ist und die immunitätserhöhenden Eigenschaften von dem undenaturierten Zustand der in der Molke enthaltenen Proteine abhängig sind.

5. Molkeprotein-Zusammensetzung gemäß Anspruch 4,
**dadurch gekennzeichnet,**
daß das Molkeproteinkonzentrat aus der von Rindern und/oder Ziegen und/oder Schafen und/oder Menschen stammenden Molkeproteinkonzentraten gebildeten Gruppe ausgewählt ist.

6. Molkeprotein-Zusammensetzung gemäß den Ansprüchen 4 oder 5,
**dadurch gekennzeichnet,**
daß das Molkeproteinkonzentrat eine Molkeproteinisolationsmischung umfaßt, in der die Hauptproteine β-Lacto-globulin, α-Lactalbumin, Immunoglobulin und Serumalbumin sind.

7. Molkeprotein-Zusammensetzung gemäß den Ansprüchen 4 oder 5, in der das Molkeproteinkonzentrat zur therapeutischen oder prophylaktischen Verabreichung in einer Konzentration von 18 bis 28 g Molkeprotein pro 100 g Nahrung, insbesondere 20 g pro 100 g Nahrung vorliegt.

8. Molkeproteinkonzentrat gemäß den Ansprüchen 4 bis 7, wobei die biologische Aktivität des Molkeproteinkonzentrats weiterhin abhängig ist vom Aminosäure-und Kleinpeptid-Profil, das aus der Verteilung der angereicherten Fraktion und/oder Fraktionen resultiert.

9. Molkeprotein-Zusammensetzung gemäß einem jeden der vorangehenden Ansprüche, enthaltend Kombinationen aus den besagten Molkeproteinkonzentraten mit den Vitaminen B1 und B2 in über dem täglichen Minimalbereich liegenden Mengen.

10. Molkeprotein-Zusammensetzung gemäß Anspruch 9, wobei die Vitamingehalte bei 1,5-2,0 mg B1 und 1,5-2,0 mg B2 pro 100 g Nahrung liegen.

11. Verwendung der Molkeprotein-Zusammensetzungen gemäß einem jeden der Ansprüche 4 bis 10 in geeigneter Konzentration für die Herstellung eines Austauschstoffs für Milch oder Milchprotein.

12. Verwendung der Molkeprotein-Zusammensetzungen gemäß einem jeden der Ansprüche 4 bis 10 für die Herstellung eines Nahrungsmittels zur Vermeidung der Bereitschaft zu einer mangelhaften Absorption von Lactose.

13. Verwendung der Molkeprotein-Zusammensetzungen gemaß einem jeden der Ansprüche 4 bis 10 für die Herstellung von Austauschstoffen für oder Zusatzstoffen zu Milchprodukten für die menschliche oder tierische Ernährung.

14. Verwendung der Molkeprotein-Zusammensetzungen gemäß einem jeden der Ansprüche 4 bis 10 für die Herstellung von Nahrungsmitteln oder Nahrungsmittelzusätzen, die an spezifische Ernährungserfordernisse angepaßt sind.

15. Verwendung der Molkeprotein-Zusammensetzungen gemäß Anspruch 4 für die Herstellung von einem Produkt zur therapeutischen Anwendung bei einer spezifischen Diätintervention.

16. Verwendung der Molkeprotein-Zusammensetzungen gemäß den Ansprüchen 4 bis 10 für die Herstellung von einem Diät-Nahrungsmittel mit erhöhter biologischer Aktivität in Kombination mit einem neutralen Träger zur oralen Verabreichung.

17. Verwendung der Molkeprotein-Zusammensetzungen gemäß den Ansprüchen 4 bis 10 für die Herstellung von einem Vorsorge-Intensivnahrungsmittel mit erhöhter biologischer Aktivität in Kombination mit einem neutralen Träger zur oralen Verabreichung.

18. Verwendung der Molkeprotein-Zusammensetzungen gemäß den Ansprüchen 4 bis 10 für die Herstellung von einem pharmazeutischen Mittel mit erhöhter biologischer Aktivität in Kombination mit einem neutralen Träger zur oralen Verabreichung.

## Revendications

1. Procédé de production d'une composition de protéines de lactosérum biologiquement active ayant une concentration appropriée de concentré protéique de lactosérum non-détanuré comportant les étapes consistant à :
a) immédiatement après la traite, refroidir le lait à une température située dans la plage allant de 2°C à 10°C, de manière spécifique à 4°C, et enlever les composants étrangers,
b) après un autre nettoyage du lait, faire précipiter le caillé en réduisant le pH à environ 4,6 à l'aide d'acide lactique, initialement à 20°C,
c) ajouter de la présure et élever la température à environ 30°C pendant 20 minutes pour favoriser l'expulsion de lactosérum à partir du caillé, en permettant l'agitation dans une cuve pour y parvenir à faible vitesse,
d) irradier et séparer le lactosérum, et
e) effectuer une première ultrafiltration du lactosérum en utilisant une membrane ayant un poids moléculaire d'exclusion d'à peu près 10 000 ou moins,
caractérisé en ce que la fraction de lactosérum destinée à être utilisée pour la production ultérieure de concentré protéique de lactosérum est maintenue à une température suffisamment basse pendant tout le processus pour minimiser la dénaturation de la protéine et du matériel à partir duquel elle est dérivée, est agitée lentement pour maintenir une solubilité élevée pour éviter une précipitation des protéines avec le caillé à l'étape c) et minimiser la dénaturation des protéines et en ce que ladite ultrafiltration est exécutée dans une ligne de production comportant jusqu'à 20 modules du type cadre supportant un grand nombre dedites membranes, aboutissant à un concentré final de protéines non-dénaturées dans la matière sèche dans lequel l'ultrafiltration est exécutée à une température située dans la plage allant de 4°C à 20°C, de manière spécifique à 4°C.

2. Procédé selon la revendication 1, dans lequel le procédé est mis en oeuvre principalement à une température en dessous de 10°C.

3. Procédé de production d'un concentré protéique de lactosérum non-dénature selon la revendication 1, caractérisé en ce que le concentré protéique de lactosérum est après ultrafiltration exempt de lactose, de sels et d'eau.

4. Concentré protéique de lactosérum sous forme sèche pouvant être obtenu conformément aux revendications 1 à 3, destiné à être utilisé en tant que médicament, dans lequel le concentré protéique de lactosérum a des propriétés de renforcement de l'immunité instables à chaud et insensibles à la digestion pancréatique, et dans le lequel les propriétés renforçant l'immunité sont fonction de l'état non-dénaturé des protéines contenues dans le lactosérum.

5. Composition de protéines de lactosérum selon la revendication 4, dans laquelle le concentré protéique de lactosérum est un concentré protéique de lactosérum choisi parmi le groupe constitué des concentrés protéiques de lactosérum bovin, et/ou caprin, et/ou ovin, et/ou humain.

6. Composition de protéines de lactosérum selon les revendications 4 et 5, dans laquelle le concentré protéique de lactosérum est constitué d'un mélange d'isolats de protéines de lactosérum dans lequel les protéines principales sont la β-lactoglobuline, l'α-lactalbumine, l'immunoglobuline et l'albumine du sérum.

7. Composition de protéines de lactosérum selon l'une quelconque des revendications 4 à 6, dans laquelle le concentré protéique de lactosérum est destiné à une administration thérapeutique ou prophylactique selon une quantité de 18 à 28 g de protéines de lactosérum/100 g d'aliments, de manière spécifique 20 g de protéines de lactosérum/100 g d'aliments.

8. Conentré protéique de lactosérum selon l'une quelconque des revendications 4 à 7, dans lequel l'activité biologique du concentré protéique de lactosérum est en outre fonction de la configuration de l'acide aminé et d'un petit peptide résultant de la contribution de la fraction renforcée et/ou des fractions renforcées.

9. Composition de protéines de lactosérum selon l'une quelconque des revendications précédentes, comportant la combinaison dudit concentré protéique de lactosérum avec des vitamines B₁ et B₂ selon des quantité destinées à l'administration dépassant les besoins quotidiens minima.

10. Composition de protéines de lactosérum selon la revendications 9, dans laquelle les quantités de vitamines sont de 1,5 à 2,0 mg pour B₁ et de 1,5 à 2,0 mg pour B₂/100 g d'aliments.

11. Utilisation de compositions de protéines de lactosérum selon l'une quelconque des revendications 4 à 10, selon une concentration appropriée pour la fabrication d'un substitut du lait ou d'une protéine du lait.

12. Utilisation de compositions de protéines de lactosérum selon l'une quelconque des revendications 4 à 10 pour la fabrication d'un aliment approprié pour éviter la tendance à une mauvaise absorption du lactose.

13. Utilisation de compositions de protéines de lactosérum selon l'une quelconque des revendications 4 à 10 pour la fabrication de substituts ou de compléments des produits quotidiens destinés à l'alimentation humaine ou à l'alimentation animale.

14. Utilisation de compositions de protéines de lactosérum selon l'une quelconque des revendications 4 à 10 pour la fabrication de nourriture ou de supplément de nourriture adoptés pour des impératifs spécifiques de nutrition.

15. Utilisation de compositions de protéines de lactosérum selon la revendication 4 pour la fabrication d'un produit ayant un objectif thérapeutique par l'intervention d'un régime spécifique.

16. Utilisation de compositions de protéines de lactosérum selon l'une quelconque des revendications 4 à 10 en combinaison avec un porteur neutre adapté à alimentation orale, pour la fabrication d'un aliment de régime ayant une activité biologique renforcée.

17. Utilisation de compositions de protéines de lactosérum selon l'une quelconque des revendications 4 à 10, en combinaison avec un porteur neutre adapté à une alimentation orale, pour la fabrication d'un aliment de sons intensifs ayant une activité biologique renforcée.

18. Utilisation de compositions de protéines de lactosérum selon l'une quelconque des revendications 4 à 10, en combinaison avec un porteur neutre adapté à une alimentation orale, pour la fabrication d'une composition pharmaceutique ayant une activité biologique renforcée.
